# EUROPEAN PATENT SPECIFICATION

(11) **EP 2 179 060 B1**
(45) Date of publication and mention of the grant of the patent: **27.11.2013**
(21) Application number: 08796304.7
(22) Date of filing: 18.07.2008
(51) Int. Cl.: C12Q 1/68

(54) **DIFFERENTIAL EXPRESSION OF MICRORNAS IN NONFAILING VERSUS FAILING HUMAN HEARTS**
DIFFERENZIELLE EXPRESSION VON microRNA BEI FUNKTIONIERENDEN IM VERGLEICH ZU NICHTFUNKTIONIERENDEN MENSCHLICHEN HERZEN
EXPRESSION DIFFÉRENTIELLE DE MICROARN DE COEURS HUMAINS NON INSUFFISANTS CONTRE DES COEURS HUMAINS INSUFFISANTS

(30) Priority: 18.07.2007 US 950565 P
(43) Date of publication of application: 28.04.2010
(73) Proprietor: The Regents of the University of Colorado, Denver, CO 80203 (US)
(72) Inventor: PORT, Jonathan David, Denver, CO 80210 (US); SUCHAROV, Carmen, Superior, CO 80027 (US); BRISTOW, Michael R., Cherry Hills Village, CO 80113 (US)
(74) Representative: HOFFMANN EITLE
(86) International application number: PCT/US2008/070508
(87) International publication number: WO 2009/012468

(56) References cited:
- WO-A2-2006/119266
- WO-A2-2008/043521
- US-A1- 2003 096 782
- US-A1- 2004 152 088
- US-A1- 2007 161 004
- VAN ROOIJ E ET AL: "A signature pattern of stress-responsive microRNAs that can evoke cardiac hyperthrophy and heart failure" PROCEEDINGS OF THE NATIONAL ACADEMY OF SCIENCES OF THE UNITED STATES (PNAS), NATIONAL ACADEMY OF SCIENCE, US LNKD- DOI:10.1073/PNAS.0608791103, vol. 103, no. 48, 28 November 2006 (2006-11-28), pages 18255-18260, XP002563455 ISSN: 0027-8424 [retrieved on 2006-11-15]
- THUM THOMAS ET AL: "MicroRNAs in the human heart: a clue to fetal gene reprogramming in heart failure." CIRCULATION 17 JUL 2007 LNKD- PUBMED:17606841, vol. 116, no. 3, 17 July 2007 (2007-07-17) , pages 258-267, XP002477098 ISSN: 1524-4539
- DATABASE Geneseq [Online] 13 July 2006 (2006-07-13), "Human mir-199a-1 microRNA, SEQ ID NO:98." XP002608646 retrieved from EBI accession no. GSN:AEH25514 Database accession no. AEH25514
- DATABASE Geneseq [Online] 14 June 2007 (2007-06-14), "LNA probe hsa-miR199a/LNA used to detect conserved vertebrate miRNAs." XP002608647 retrieved from EBI accession no. GSN:AFO07977 Database accession no. AFO07977
- Kittleson Michelle Maya: "Gene expression profiling in ischemic and nonischemic cardiomyopathy" 3 August 2006 (2006-08-03), XP002608648 Retrieved from the Internet: URL:https://jscholarship.library.jhu.edu/h andle/1774.2/865 [retrieved on 2010-11-09]
- LUI ET AL.: 'Patterns of Known and Novel Small RNAs in Human Cervical Cancer.' CANCER RESEARCH vol. 67, no. 13, 01 July 2007, pages 6031 - 60432, XP002544106

## Description

### Cross-Reference to Related Applications

This application claims the benefit of U.S. Provisional Application No. 60/950,565, filed July 18, 2007.

### Background of the Invention

Heart failure is a pathophysiological state in which the heart is unable to pump sufficient blood to meet the nutrition and oxygen requirement of metabolizing tissues or cells. It is a major complication in many heart diseases. Adults over the age of 40 have an estimated 21 % lifetime risk of developing heart failure (Lloyd-Jones et al., 2002, Circulation 106, 3068-72), a condition responsible for more hospitalizations than all forms of cancer combined (American Heart Association. Heart Disease and Stroke Statistics--2003 Update).

Heart failure is a general term that describes the final common endpoint of many disease processes. The most common cause of heart failure is coronary artery disease, which can lead to a myocardial infarction (heart attack), often resulting in death of cardiac cells. In an attempt to compensate, the heart must then perform the same work with the remaining viable tissue. Chronic obstructive coronary artery disease can also cause heart failure in the absence of myocardial infarction. Valve disease or high blood pressure can lead to heart failure by increasing the workload of the heart. Less frequent causes of heart failure, which primarily involve cardiac muscle, are classed as cardiomyopathy (although this term is sometimes used more generally to cover any cause of heart failure). The most well characterized of these disorders are a group of single gene disorders of the sarcomere which frequently result in a "hypertrophic cardiomyopathy" phenotype (in fact, a misnomer as many patients have no hypertrophy, i.e. there is variable phenotypic penetrance). In contrast, all patients with "dilated cardiomyopathy" have dilated thin walled ventricles. The genetics of this condition have yet to be characterized and are likely to be multifacforial , but in many cases non-genetic causes can contribute significantly (e.g. infections, alcohol, chemotherapeutic agents). Where no readily identifiable cause is found, the diagnosis used is idiopathic dilated cardiomyopathy (generally a diagnosis of exclusion).

A variety of pathophysiological changes occur in the heart as heart failure develops. In response to increased work load *in vivo,* the heart frequently increases in size (cardiac hypertrophy) as a direct result but secondary to cardiac muscle cell hypertrophy (i.e., an increase in cell size in the absence of cell division). At the cellular and molecular levels, cardiac hypertrophy is characterized by increased expression of contractile proteins and activation of various signaling pathways whose role in the pathophysiology of heart failure remains incompletely understood.

Current treatments for heart failure include pharmacological methods, devices such as the ventricular assist device (VAD), cardiac resynchronization therapy (CRT), and heart transplantation. Pharmacological approaches include but are not limited to the use of inotropic agents (i.e., compounds that increase cardiac contractility), neurohumoral blockers (e.g., beta-blockers, angiotensin converting enzyme inhibitors), aldosterone antagonists, diuretics, and vasodilators. However, none of these agents is fully effective either alone or in combination. Availability of transplants is highly limited, and since many individuals suffering from heart failure are in poor health, they are frequently not good surgical candidates. For these reasons, heart failure remains a major cause of morbidity and mortality, particularly in the developed world. In addition, as indicated above, it can be difficult to determine the precise etiology of heart failure, a factor impeding the development of more specific therapies. In addition, there is a general lack of diagnostic techniques at the molecular level. Kittleson Michelle Maya ("Gene expression profiling in ischemic and nonischemic cardiomyopathy", 2006-08-03, URL:https://jscholarship.library.jhu.edu/handle/1774.2/865) discloses differential gene expression in ischemic and nonischemic cardiomyopathy. Thus, there is a need in the art for the discovery of additional diagnostic markers and pharmacological targets for the development of new therapeutic approaches for the treatment of heart failure. In addition, there is a need in the art for improved techniques for evaluating the severity of heart failure and its response to treatment, both current and yet to be described. The present invention addresses the foregoing needs, among others.

Cardiac hypertrophy and its potential for progression to heart failure is a common pathological response to a number of cardiovascular diseases including hypertension, ischemic heart disease, valvular diseases, and endocrine disorders. Cardiac hypertrophy refers to a thickening of the heart muscle (myocardium) which may result in obstruction of blood flow and/or a decrease in cardiac function (diastolic relaxation). Cardiac hypertrophy can occur when the heart is stressed as a consequence of either normal physiological processes (rigorous exercise), or pathophysiological processes such as hypertension, valvular stenosis or insufficiency, ischemia, or metabolic disorders; secondarly, cardiac hypertrophy is frequently accompanied by programmed cell death (apoptosis) and/or tissue necrosis. Given that cardiac cells are for the most part incapable of dividing, cells grow larger in an attempt to compensate; initially, this compensation is helpful, but excessive hypertrophy can progressively activate a number of other pathophysiolical cascades that result in progressive cardiac dysfunction and ultimately to decompensated heart failure. Since cardiac hypertrophy can be an independent risk factor, additive to other heart-disease risk factors, such as high blood pressure, hypertrophy may or may not regress when high blood pressure is treated. It would therefore be beneficial to have a direct treatment for cardiac hypertrophy.

MicroRNAs (miRNAs) are a recently discovered class of regulatory RNAs that post-transcriptionally regulate gene expression. miRNAs are evolutionarily conserved, small non-coding RNA molecules of approximately 18 to 25 nucleotides in length. miRNAs base pair with specific "target" mRNAs and in doing so inhibit translation or promote mRNA degradation (Bartel, 2004 Cell, 116, 281-297).

miRNAs have been reported to be involved in the development of organisms (Ambros, Cell 113: 673-676). miRNAs are differentially expressed in numerous tissues (Xu et al., 2003 Curr. Biol. 13:790-795; Landgraf et al., 2007 Cell 129:1401-14). miRNAs have also been reported to be involved in viral infection processes (Pfeffer et al., 2004 Science 304: 734-736), and associated with oncogenesis (Calin, et al., 2004 Proc. Natl. Acad. Sci. USA 101: 2999-3004; Calin et al., 2002, Proc. Natl. Acad. Sci. USA 99(24): 15524-15529). Although certain miRNAs are expressed at a high abundance in the heart, the roles of these miRNAs in various cardiac diseases remains to be defined.

### Summary of the Invention

The invention provides an *in vitro* method of diagnosing ischemic cardiomyopathy in a subject, the method comprising:
a. determining the level of expresison of one or more miRNAs in a biological sample, from the subject, wherein said one or more miRNAs is selected from the group consisting of hsa-miR-199a (SEQ ID NO: 25), hsa-miR-195 (SEQ ID NO: 4), hsa-miR-100 (SEQ ID NO: 7), hsa-miR-342 (SEQ ID NO: 14), hsa-miR-28 (SEQ ID NO: 24), hsa-miR-26b (SEQ ID NO: 30), hsa-miR-92a (SEQ ID NO: 5), hsa-miR-221 (SEQ ID NO: 17), hsa-miR-486 (SEQ ID NO: 11), hsa-miR-133a (SEQ ID NO: 15), hsa-miR-150 (SEQ ID NO: 12), hsa-miR-422b (SEQ ID NO: 16), hsa-let-7f (SEO ID NO: 18), hsa-let-7a (SEQ ID NO: 22), hsa-miR-1 (SEQ ID NO: 23), hsa-miR-222 (SEQ ID NO: 20), hsa-let-7c (SEQ ID NO: 28), hsa-miR-133b (SEQ ID NO: 19), hsa-miR-224 (SEQ ID NO: 21), hsa-miR-484 (SEQ ID NO: 29), hsa-let-7d (SEQ ID NO: 31); and
b. comparing the level of expression of one or more miRNAs in said biological sample to the level of one or more miRNAs in a standard sample,
wherein a difference in the level of expression in at least one or more miRNAs in said biological sample compared to the level of one or more miRNAs in said standard sample is diagnostic for ischemic cardiomyopathy.

In one embodiment of the invention, an increase in the level of any one of hsa-miR-199a (SEQ ID NO: 25), hsa-miR-195 (SEQ ID NO: 4), hsa-miR-100 (SEQ ID NO: 7), hsa-miR-342 (SEQ ID NO: 14), hsa-miR-26b (SEQ ID NO: 30),and hsa-miR-28 (SEQ ID NO: 24) is diagnostic of ischemic cardiomyopathy.

In another embodiment of the invention, a decrease in the level of any one of hsa-miR-92a (SEQ ID NO: 5), hsa-miR-221 (SEQ ID NO: 17), hsa-miR-486 (SEQ ID NO: 11), hsa-miR-133a (SEQ ID NO: 15), hsa-miR-150 (SEQ ID NO: 12), hsa-miR-422b (SEQ ID NO: 16), hsa-let-7f (SEQ ID NO: 18), hsa-let-7a (SEQ ID NO: 22), hsa-miR-1 (SEQ ID NO: 23), hsa-miR-222 (SEQ ID NO: 20), hsa-let-7c (SEQ ID NO: 28), hsa-miR-133b (SEQ ID NO: 19), hsa-miR-224 (SEQ ID NO: 21), hsa-miR-484 (SEQ ID NO: 29), and hsa-let-7d (SEQ ID NO: 31) is diagnostic of ischemic cardiomyopathy.

The present disclosure also provides a method of diagnosing or prognosing heart failure in a subject. In one aspect of the disclosure, the method comprises determining the level of expression of one or more miRNAs in a biological sample from the subject; and comparing the level of expression of one or more miRNAs in said biological sample to the level of one or more miRNAs in a standard sample; wherein a difference in the level of expression in at least one or more miRNAs in said biological sample compared to the level of one or more miRNAs in said standard sample is diagnostic or prognostic for heart failure.

In another aspect of the disclosure, one or more miRNAs for use in the method of diagnosing or prognosing heart failure is selected from hsa-miR-542-5p (SEQ ID NO: 1), hsa-miR-125b (SEQ ID NO: 2), hsa-miR-197 (SEQ ID NO: 3), hsa-miR-195 (SEQ ID NO: 4), hsa-miR-92a (SEQ ID NO: 5), hsa-miR-139 (SEQ ID NO: 6), hsa-miR-100 (SEQ ID NO: 7), hsa-miR-483 (SEQ ID NO: 8), hsa-miR-22 (SEQ ID NO: 9), hsa-miR-23a (SEQ ID NO: 10), hsa-miR-486 (SEQ ID NO: 11), hsa-miR-150 (SEQ ID NO: 12), hsa-miR-30c (SEQ ID NO: 13), hsa-miR-342 (SEQ ID NO: 14), hsa-miR-133a (SEQ ID NO: 15), hsa-miR-422b (also known as miR-378; SEQ ID NO: 16), hsa-miR-221 (SEQ ID NO: 17), hsa-let-7f (SEQ ID NO: 18), hsa-miR-133b (SEQ ID NO: 19), hsa-miR-222 (SEQ ID NO: 20), hsa-miR-224 (SEQ ID NO: 21), hsa-let-7a (SEQ ID NO: 22), hsa-miR-1 (SEQ ID NO: 23), hsa-miR-28 (SEQ ID NO: 24), hsa-miR-199a (SEQ ID NO: 25), hsa-miR-181b (SEQ ID NO: 26), hsa-miR-20a (SEQ ID NO: 27), hsa-let-7c (SEQ ID NO: 28), hsa-miR-484 (SEQ ID NO: 29), hsa-miR-26b (SEQ ID NO: 30), hsa-let-7d (SEQ ID NO: 31), hsa-miR-10b (SEQ ID NO: 32), hsa-miR-382 (SEQ ID NO: 33) hsa-miR-92b (SEQ ID NO: 48), hsa-let-7b (SEQ ID NO: 49), hsa-let-7e (SEQ ID NO: 50), and hsa-let-7g (SEQ ID NO:51).

In another aspect of the disclosure, one or more miRNAs for use in the method of diagnosing or prognosing heart failure is selected from: hsa-miR-125b (SEQ ID NO: 2), hsa-miR-195 (SEQ ID NO: 4), hsa-miR-92a (SEQ ID NO: 5), hsa-miR-100 (SEQ ID NO: 7), hsa-miR-22 (SEQ ID NO: 9), hsa-miR-486 (SEQ ID NO: 11), hsa-miR-150 (SEQ ID NO: 12), hsa-miR-30c (SEQ ID NO: 13), hsa-miR-342 (SEQ ID NO: 14), hsa-miR-133a (SEQ ID NO: 15), hsa-miR-221 (SEQ ID NO: 17), hsa-let-7f (SEQ ID NO: 18), hsa-miR-133b (SEQ ID NO: 19), hsa-miR-222 (SEQ ID NO: 20), hsa-let-7a (SEQ ID NO: 22), hsa-miR-1 (SEQ ID NO: 23), hsa-miR-28 (SEQ ID NO: 24), and hsa-miR-199a (SEQ ID NO: 25).

In one aspect, one or more miRNAs for use in the method of diagnosing or prognosing heart failure is selected from hsa-miR-195 (SEQ ID NO: 4), hsa-miR-92a (SEQ ID NO: 5), hsa-miR-100 (SEQ ID NO: 7), hsa-miR-486 (SEQ ID NO: 11), hsa-miR-150 (SEQ ID NO: 12), and hsa-miR-422b (also known as miR-378; SEQ ID NO: 16).

In yet another aspect of the disclosure, the type of heart failure is diagnosed based upon the level of one or more miRNAs in said biological sample compared to the level of one or more miRNAs in said standard sample, wherein the level of one or more miRNAs is diagnostic for ischemic cardiomyopathy or idiopathic dilated cardiomyopathy. Preferably, one or more miRNAs diagnostic for ischemic cardiomyopathy or idiopathic dilated cardiomyopathy is selected from: hsa-miR-125b (SEQ ID NO: 2), hsa-miR-22 (SEQ ID NO: 9), hsa-miR-150 (SEQ ID NO: 12), hsa-miR-30c (SEQ ID NO: 13), hsa-miR-342 (SEQ ID NO: 14), hsa-miR-133a (SEQ ID NO: 15), hsa- miR-422b (also known as miR-378; SEQ ID NO: 16), hsa-let-7f (SEQ ID NO: 18), hsa-miR-133b (SEQ ID NO: 19), hsa-miR-222 (SEQ ID NO: 20), hsa-let-7a (SEQ ID NO: 22), hsa-miR-1 (SEQ ID NO: 23), hsa-miR-28 (SEQ ID NO: 24), and hsa-miR-199a (SEQ ID NO: 25).

The present disclosure also provides a method of treating or preventing heart failure or a disease or condition associated with heart failure in a subject. In one aspect, the method comprises administering a therapeutically effective amount of a miRNA mimic or a miRNA inhibitor to heart cells of the subject.

In another aspect of the disclosure, the miRNA mimic or miRNA inhibitor used in a method of treating or preventing heart failure or a disease or condition associated with heart failure is a mimic or inhibitor of one or more of the miRNAs selected from the group consisting of: hsa-miR-542-5p (SEQ ID NO: 1), hsa-miR-125b (SEQ ID NO: 2), hsa-miR-197 (SEQ ID NO: 3), hsa-miR-195 (SEQ ID NO: 4), hsa-miR-92a (SEQ ID NO: 5), hsa-miR-139 (SEQ ID NO: 6), hsa-miR-100 (SEQ ID NO: 7), hsa-miR-483 (SEQ ID NO: 8), hsa-miR-22 (SEQ ID NO: 9), hsa-miR-23a (SEQ ID NO: 10), hsa-miR-486 (SEQ ID NO: 11), hsa-miR150 (SEQ ID NO: 12), hsa-miR-30c (SEQ ID NO: 13), hsa-miR-342 (SEQ ID NO: 14), hsa-miR-133a (SEQ ID NO: 15), hsa-miR-422b (also known as miR-378; SEQ ID NO: 16), hsa-miR-221 (SEQ ID NO: 17), hsa-let-7f (SEQ ID NO: 18), hsa-miR-133b (SEQ ID NO: 19), hsa-miR-222 (SEQ ID NO: 20), hsa-miR-224 (SEQ ID NO: 21), hsa-let-7a (SEQ ID NO: 22), hsa-miR-1 (SEQ ID NO: 23), hsa-miR-28 (SEQ ID NO: 24), hsa-miR-199a (SEQ ID NO: 25), hsa-miR-181b (SEQ ID NO: 26), hsa-miR-20a (SEQ ID NO: 27), hsa-let-7c (SEQ ID NO: 28), hsa-miR-484 (SEQ ID NO: 29), hsa-miR-26b (SEQ ID NO: 30), hsa-let-7d (SEQ ID NO: 31), hsa-miR-10b (SEQ ID NO: 32), hsa-miR-382 (SEQ ID NO: 33), hsa-miR-92b (SEQ ID NO: 48), hsa-let-7b (SEQ ID NO: 49), hsa-let-7e (SEQ ID NO: 50), and hsa-let-7g (SEQ ID NO: 51). In another aspect of the disclosure, the method further comprises administering to the subject a second cardiac therapy. The second cardiac therapy may include an inotropic agent, a neurohumoral blocker, an aldosterone antagonist, a diuretic, or a vasodilator.

In still another aspect of the disclosure, the method of treating or preventing heart failure or a disease or condition associated with heart failure in a subject comprises administering to the subject a pharmaceutical composition, wherein said pharmaceutical composition comprises at least one miRNA mimic or at least one miRNA inhibitor and a pharmaceutically acceptable carrier. The miRNA mimic or miRNA inhibitor may be a mimic or inhbitor of hsa-miR-542-5p (SEQ ID NO: 1), hsa-miR-125b (SEQ ID NO: 2), hsa-miR-197 (SEQ ID NO: 3), hsa-miR-195 (SEQ ID NO: 4), hsa-miR-92a (SEQ ID NO: 5), hsa-miR-139 (SEQ ID NO: 6), hsa-miR-100 (SEQ ID NO: 7), hsa-miR-483 (SEQ ID NO: 8), hsa-miR-22 (SEQ ID NO: 9), hsa-miR-23a (SEQ ID NO: 10), hsa-miR-486 (SEQ ID NO: 11), hsa-miR150 (SEQ ID NO: 12), hsa-miR-30c (SEQ ID NO: 13), hsa-miR-342 (SEQ ID NO: 14), hsa-miR-133a (SEQ ID NO: 15), hsa-miR-422b (also known as miR-378; SEQ ID NO: 16), hsa-miR-221 (SEQ ID NO: 17), hsa-let-7f (SEQ ID NO: 18), hsa-miR-133b (SEQ ID NO: 19), hsa-miR-222 (SEQ ID NO: 20), hsa-miR-224 (SEQ ID NO: 21), hsa-let-7a (SEQ ID NO: 22), hsa-miR-1 (SEQ ID NO: 23), hsa-miR-28 (SEQ ID NO: 24), hsa-miR-199a (SEQ ID NO: 25), hsa-miR-181b (SEQ ID NO: 26), hsa-miR-20a (SEQ ID NO: 27), hsa-let-7c (SEQ ID NO: 28), hsa-miR-484 (SEQ ID NO: 29), hsa-miR-26b (SEQ ID NO: 30), hsa-let-7d (SEQ ID NO: 31), hsa-miR-10b (SEQ ID NO: 32), hsa-miR-382 (SEQ ID NO: 33), hsa-miR-92b (SEQ ID NO: 48), hsa-let-7b (SEQ ID NO: 49), hsa-let-7e (SEQ ID NO: 50), or hsa-let-7g (SEQ ID NO: 51). In another aspect of the disclosure, the pharmaceutical composition further comprises one or more of an inotropic agent, a neurohumoral blocker, an aldosterone antagonist, a diuretic, and a vasodilator.

The disclosure also provides a method of regulating reactivation of the fetal cardiac gene program (FGP) in a cell. In one aspect, the method comprises providing a cardiac cell with one or more miRNA inhibitors or miRNA mimics, wherein the one or more miRNA inhibitors or miRNA mimics modulates expression of at least one marker of the fetal cardiac gene program. The one or more miRNA inhibitors or miRNA mimics may be mimics or inhibitors of one or more miRNAs selected from the group consisting of hsa-miR-542-5p (SEQ ID NO: 1), hsa-miR-125b (SEQ ID NO: 2), hsa-miR-197 (SEQ ID NO: 3), hsa-miR-195 (SEQ ID NO: 4), hsa-miR-92a (SEQ ID NO: 5), hsa-miR-139 (SEQ ID NO: 6), hsa-miR-100 (SEQ ID NO: 7), hsa-miR-483 (SEQ ID NO: 8), hsa-miR-22 (SEQ ID NO: 9), hsa-miR-23a (SEQ ID NO: 10), hsa-miR-486 (SEQ ID NO: 11), hsa-miR150 (SEQ ID NO: 12), hsa-miR-30c (SEQ ID NO: 13), hsa-miR-342 (SEQ ID NO: 14), hsa-miR-133a (SEQ ID NO: 15), hsa-miR-422b (also known as miR-378; SEQ ID NO: 16), hsa-miR-221 (SEQ ID NO: 17), hsa-let-7f (SEQ ID NO: 18), hsa-miR-133b (SEQ ID NO: 19), hsa-miR-222 (SEQ ID NO: 20), hsa-miR-224 (SEQ ID NO: 21), hsa-let-7a (SEQ ID NO: 22), hsa-miR-1 (SEQ ID NO: 23), hsa-miR-28 (SEQ ID NO: 24), hsa-miR-199a (SEQ ID NO: 25), hsa-miR-181b (SEQ ID NO: 26), hsa-miR-20a (SEQ ID NO: 27), hsa-let-7c (SEQ ID NO: 28), hsa-miR-484 (SEQ ID NO: 29), hsa-miR-26b (SEQ ID NO: 30), hsa-let-7d (SEQ ID NO: 31), hsa-miR-10b (SEQ ID NO: 32), hsa-miR-382 (SEQ ID NO: 33), hsa-miR-92b (SEQ ID NO: 48), hsa-let-7b (SEQ ID NO: 49), hsa-let-7e (SEQ ID NO: 50), and hsa-let-7g (SEQ ID NO: 51). In one aspect, the one or more miRNA inhibitors or miRNA mimics is a mimic or inhibitor of hsa-miR-92a (SEQ ID NO: 5), hsa-miR-100 (SEQ ID NO: 7), or hsa-miR-133b (SEQ ID NO: 19). In another aspect, the at least one marker of the fetal cardiac gene program is selected from the group consisting of αMyHC, βMyHC, ANF, BNP, SERCA2a, and skeletal α-actin.

The present disclosure also encompasses a method of monitoring treatment for heart failure in a subject. In one aspect, the method comprises obtaining a first biological sample from the subject at a first time point, wherein the first time point is prior to the start of a drug therapy protocol for heart failure; obtaining a second biological sample from the subject at a second time point, wherein the second time point is after the start of a drug therapy protocol for heart failure; processing the first biological sample and second biological sample by miRNA array analysis, mRNA array analysis and targeted proteomic expression analysis; and comparing miRNA, mRNA, and protein expression data from the first biological sample to the expression data from the second biological sample to determine changes in gene expression patterns, wherein changes in gene expression are indicative of the efficacy of the drug therapy protocol. In another aspect, the first time point and the second time point are about three to nine months apart. In another aspect, the mRNA analysis is performed by an Affymetrix protocol.

### Brief Description of the Drawings

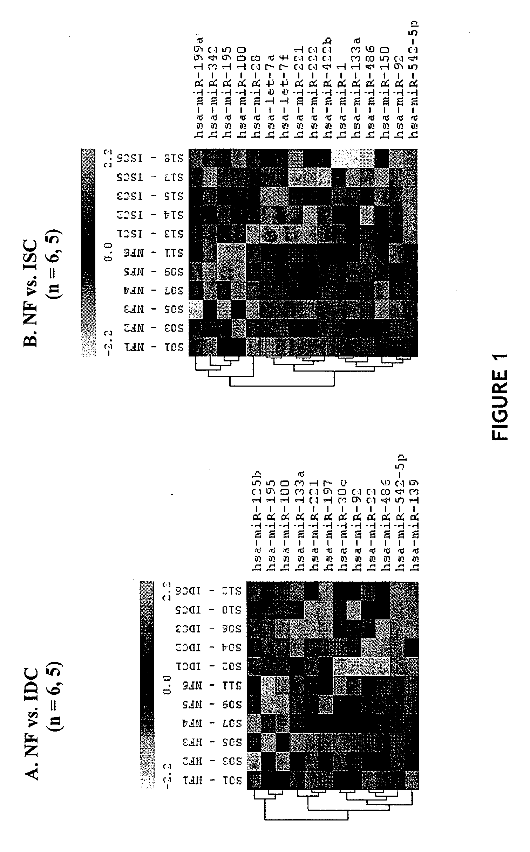

**Figure 1****.** Relative expression of miRNAs. **A.** nonfailing (NF, n=6) versus failing idiopathic dilated cardiomyopathy (IDC, n=5) hearts, and **B.** NF versus failing, ischemic cardiomyopathy (ISC, n=5) hearts. Analysis was performed on LC Sciences (Houston, TX) miRNA expression array chips corresponding to the Sanger human miRBase 9.0, a data set of 470 miRNAs. Data are represented in pseudo-thermal scale with red representing increased expression and green representing decreased expression. Only miRNAs that were significantly up or down regulated (p<0.05) are represented.

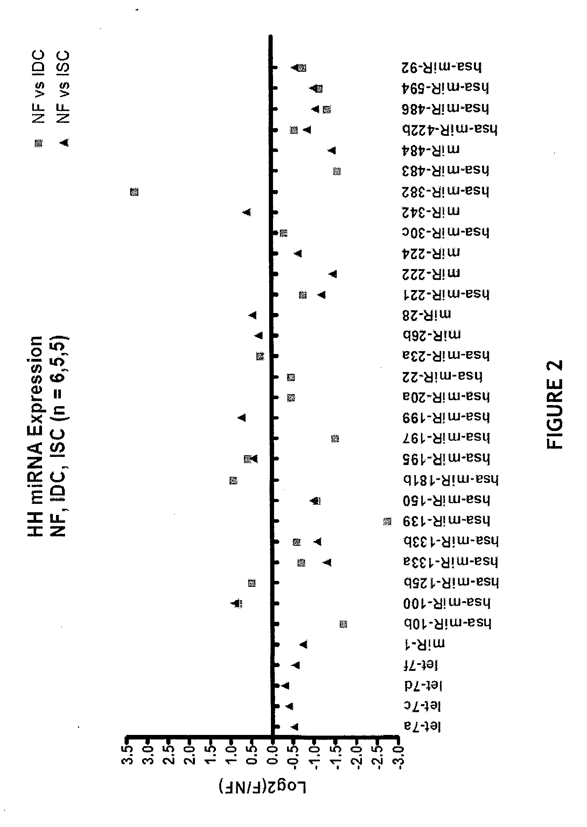

**Figure 2****.** Graphic representation of relative miRNA expression in nonfailing (NF, n=6) versus failing (IDC or ISCH, n=5,5) human hearts. Note that certain miRNAs are up or down regulated in both types of failing hearts whereas others are differentially expressed based upon type of heart failure.

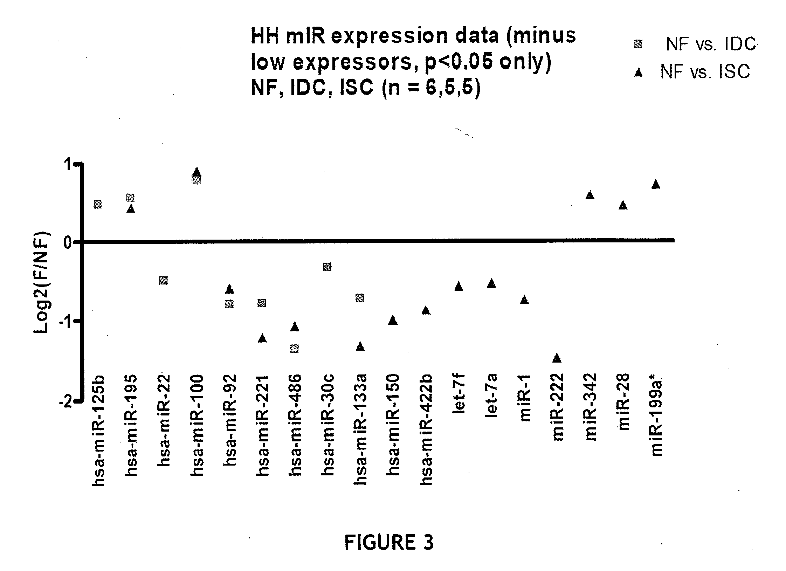

**Figure 3****.** Relative expression for the subset of miRNAs with differential expression achieving p<0.05.

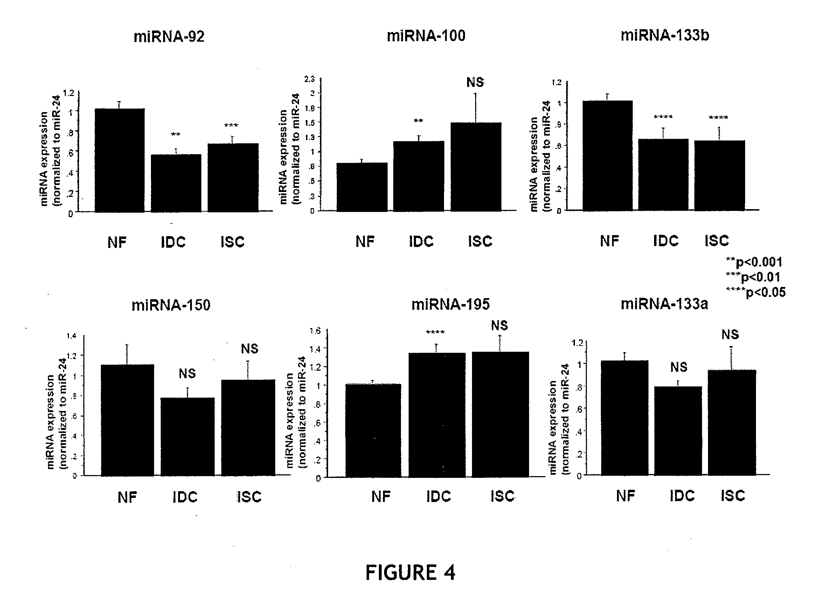

**Figure 4****.** miRNA expression levels as assessed by RT-PCR from RNA extracted from non-failing (NF), idiopathic cardiomyopathy (IDC) or ischemic cardiomyopathy (ISC) subjects. miRNA expression was normalized to that of miR-24, expression of which is constant between non-failing and failing hearts. Statistical analysis was done using ANOVA.

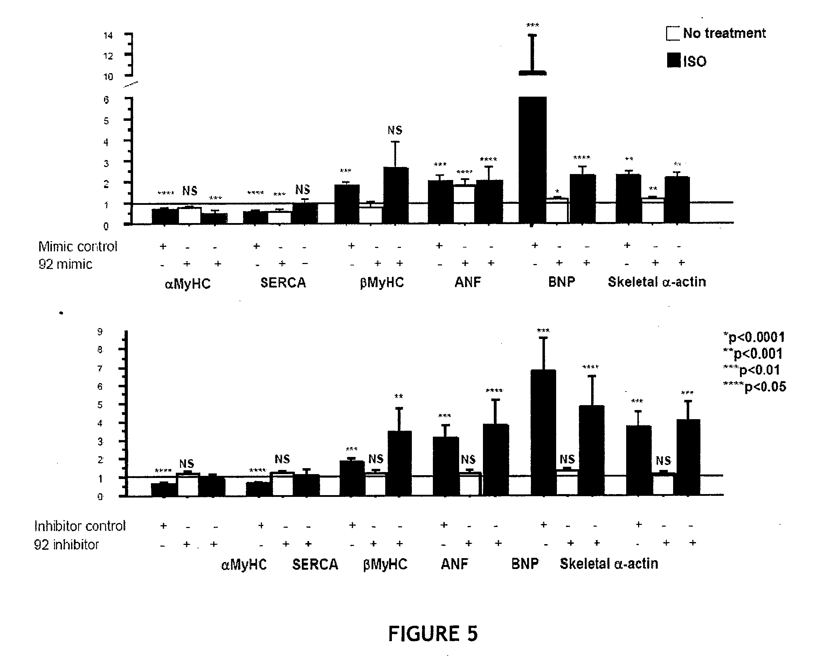

**Figure 5****.** Changes in fetal gene expression in neonatal rat ventricular myocytes transfected with miR-92 mimic or miR-92 inhibitor. Cells were treated with 10⁻⁷M isoproterenol (ISO; black bars) 24 hours after transfection and harvested 48 hours after treatment. Gene expression was measured by RT-PCR. Results were normalized to 18S rRNA expression levels and were compared to cells transfected with a control mimic or inhibitor, defined as 1 (line at 1). The graph represents an average of 3-4 individual experiments.

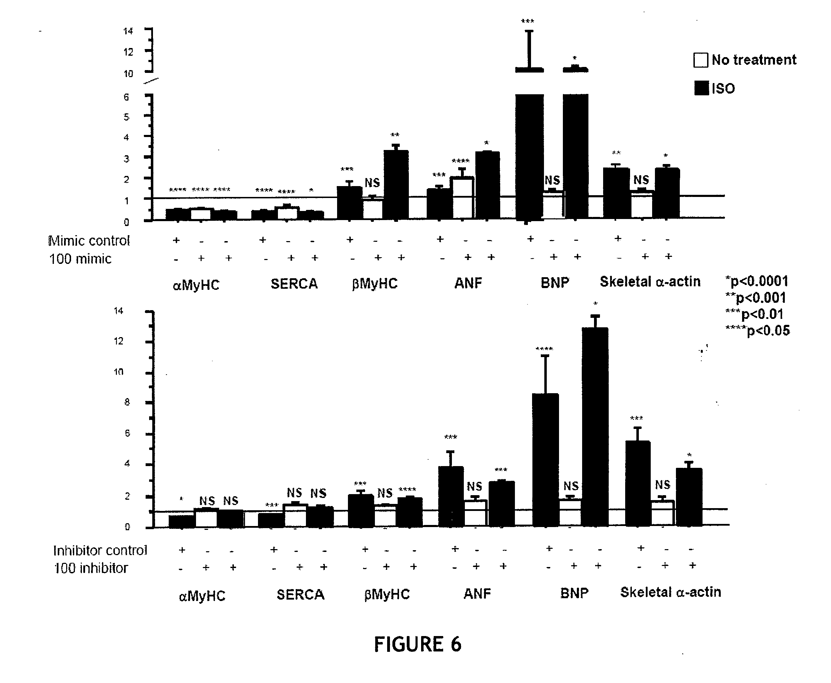

**Figure 6****.** Changes in fetal gene expression in neonatal rat ventricular myocytes transfected with miR-100 mimic or miR-100 inhibitor. Cells were treated with 10⁻⁷M isoproterenol (ISO; black bars) 24 hours after transfection and harvested 48 hours after treatment. Gene expression was measured by RT-PCR. Results were normalized to 18S rRNA expression levels and were compared to cells transfected with a control mimic or inhibitor, defined as 1 (line at 1). The graph represents an average of 3-4 individual experiments.

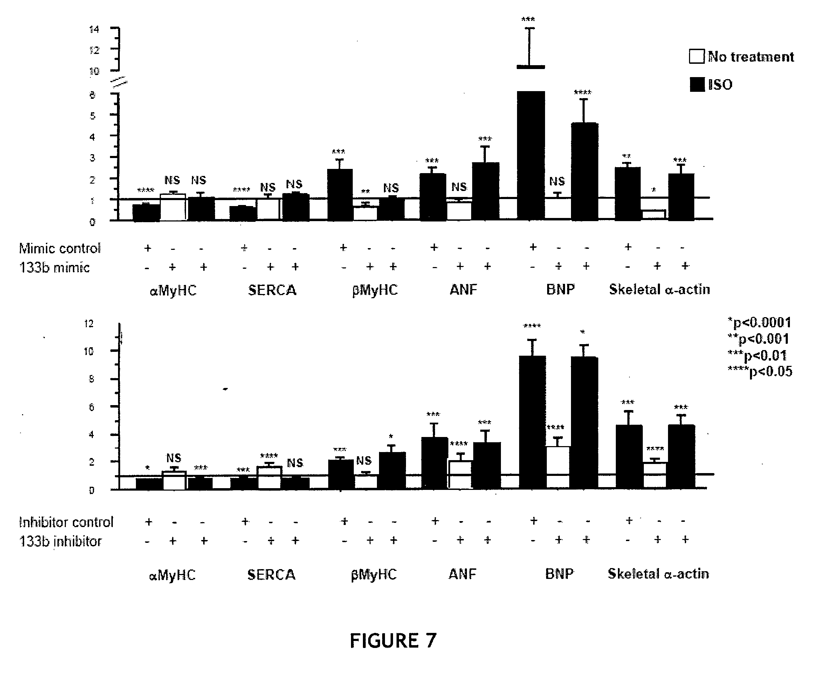

**Figure 7****.** Changes in fetal gene expression in neonatal rat ventricular myocytes transfected with miR-133b mimic or miR-133b inhibitor. Cells were treated with 10⁻⁷M isoproterenol (ISO; black bars) 24 hours after transfection and harvested 48 hours after treatment. Gene expression was measured by RT-PCR. Results were normalized to 18S rRNA expression levels and were compared to cells transfected with a control mimic or inhibitor, defined as 1 (line at 1). The graph represents an average of 3-4 individual experiments.

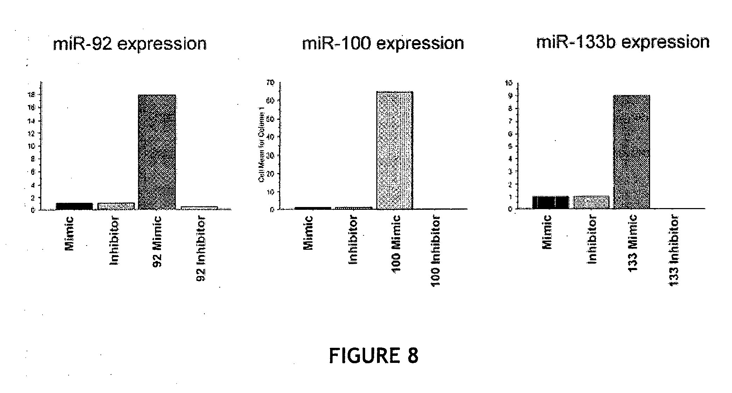

**Figure 8****.** miRNA levels upon transfection with the mimic miRNA or the inhibitor miRNA. Mimics and inhibitors for miR-92, miR-100 and miR-133b were transfected into neonate rat ventricular myocytes. Expression levels of each miRNA were assessed by RT-PCR.

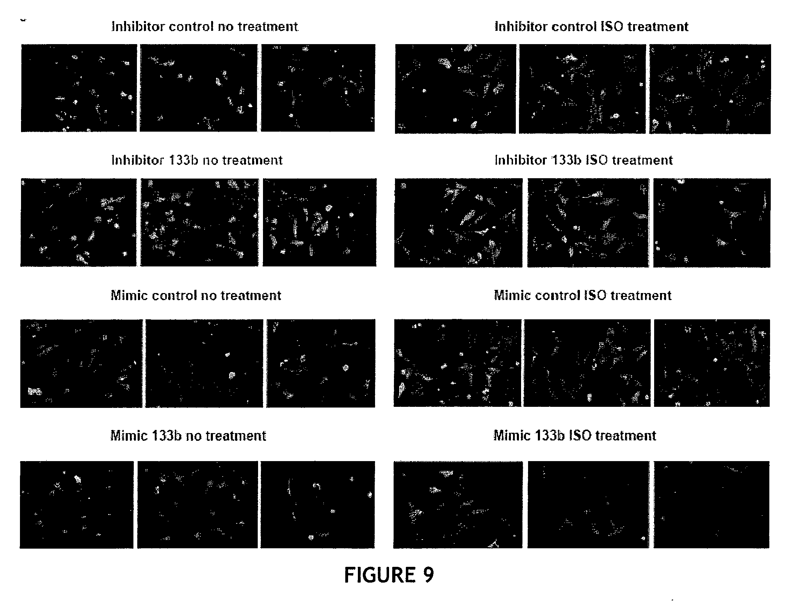

**Figure 9****.** Over-expression or down-regulation of miRNA-133b in neonatal rat ventricular myocytes regulates cellular hypertrophy. Cells were transfected with miR-133b mimic (lower panels) or miR-133b inhibitor (upper panels) and were subjected to either isoproterenol treatment (right panels) or no further treatment (left panels). The figure shows immunofluorescence with anti-actinin antibody.

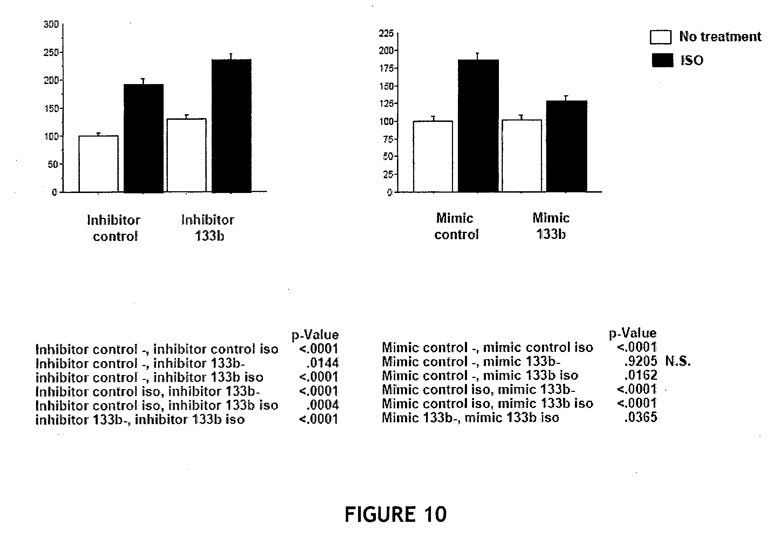

**Figure 10****.** Over-expression or down-regulation of miRNA-133b in neonatal rat ventricular myocytes regulates cellular hypertrophy. Cells were transfected with miR-133b mimic or miR-133b inhibitor and a subset of transfected cells were exposed to isoproterenol (black bars). Cell size was measured using the Image J software. A total of 30 cells from 3 different fields were measured.

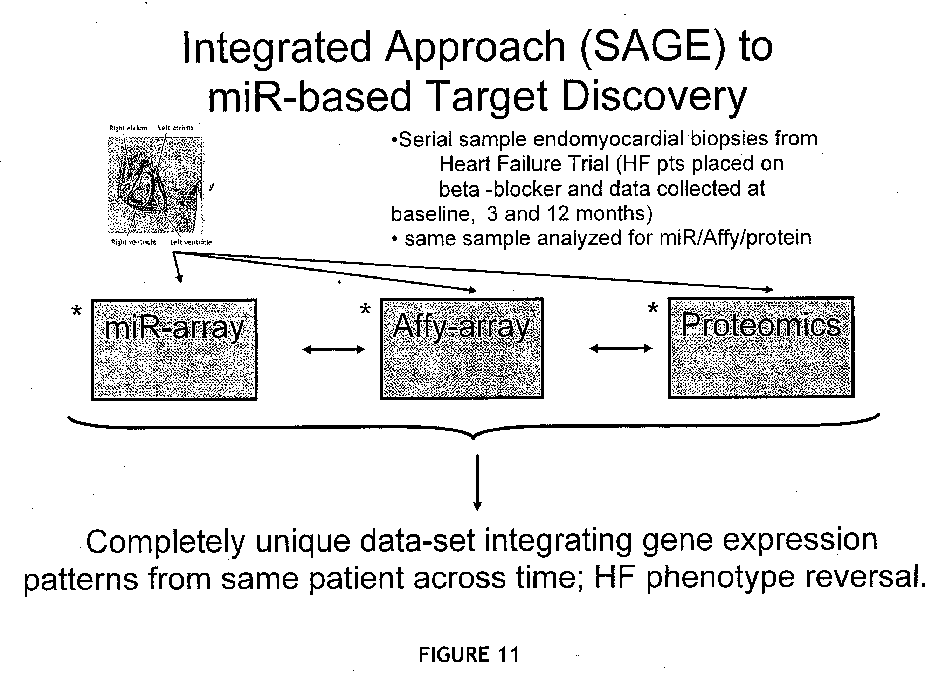

**Figure 11****.** Schematic of serial analysis of gene expression (SAGE) as an integrated approach to a method of miRNA-based target discovery. Endomyocardial biopsy from heart failure (HF) patients is used to provide biological samples for miRNA array analysis, mRNA (Afflymetrix) array analysis, and (targeted) proteomic expression analysis.

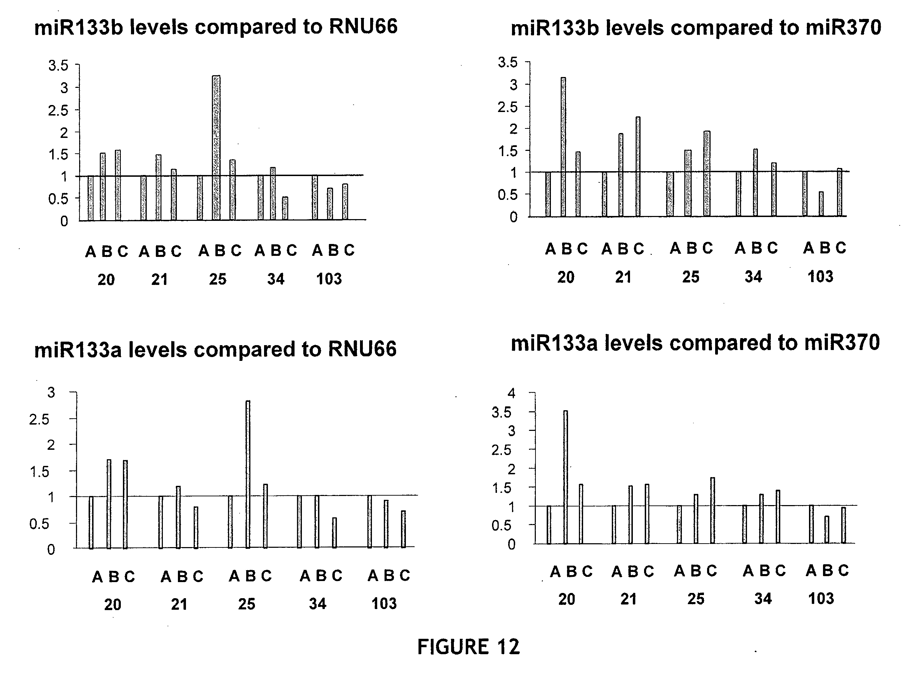

**Figure 12****.** miR-133 expression in human patients treated with β-blockers. RT-PCR analysis was performed on samples from patients treated with β-blockers. Serial biopsies were obtained at end stage heart failure (A), 3 months post-treatment (B) and 12 months post-treatment (C). The expression levels of miR-133b (top panels) and miR-133a (bottom panels) were normalized to the expression of miR-370 (right panels) and the small RNA RNU66 (left panels). Patients 20, 21, 25 and 34 responded to β-blocker treatment, while patient 103 did not respond to the treatment.

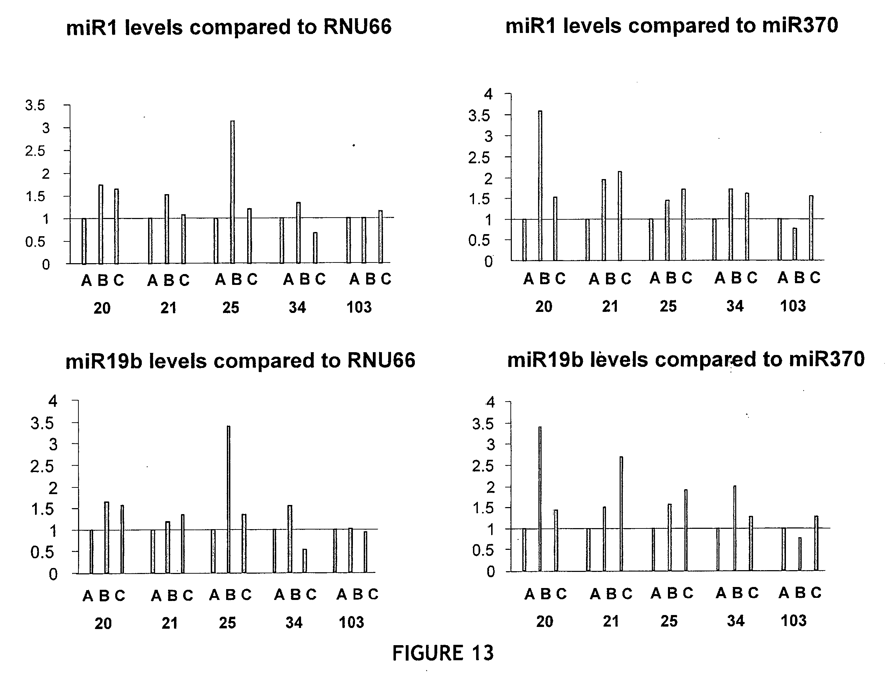

**Figure 13****.** miR-1 and miR-19b expression in human patients treated with β-blockers. RT-PCR analysis was performed on samples from patients treated with β-blockers. Serial biopsies were obtained at end stage heart failure (A), 3 months post-treatment (B) and 12 months post-treatment (C). The expression levels of miR-1 (top panels) and miR-19b (bottom panels) were normalized to the expression of miR-370 (right panels) and the small RNA RNU66 (left panels). Patients 20, 21, 25 and 34 responded to β-blocker treatment, while patient 103 did not respond to the treatment.

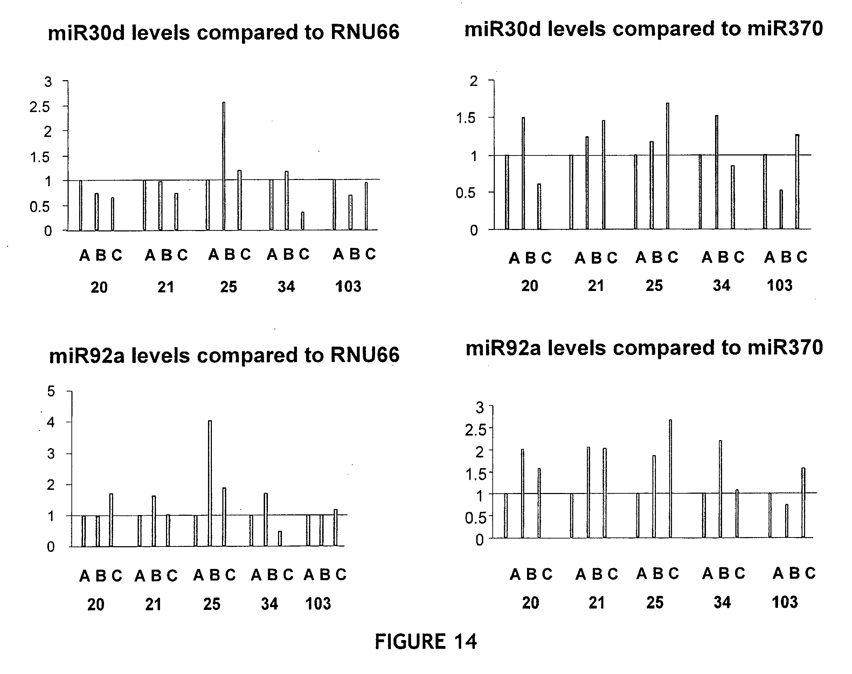

**Figure 14****.** miR-30d and miR-92a expression in human patients treated with β-blockers. RT-PCR analysis was performed on samples from patients treated with β-blockers. Serial biopsies were obtained at end stage heart failure (A), 3 months post-treatment (B) and 12 months post-treatment (C). The expression levels of miR-30d (top panels) and miR-92a (bottom panels) were normalized to the expression of miR-370 (right panels) and the small RNA RNU66 (left panels). Patients 20, 21, 25 and 34 responded to β-blocker treatment, while patient 103 did not respond to the treatment.

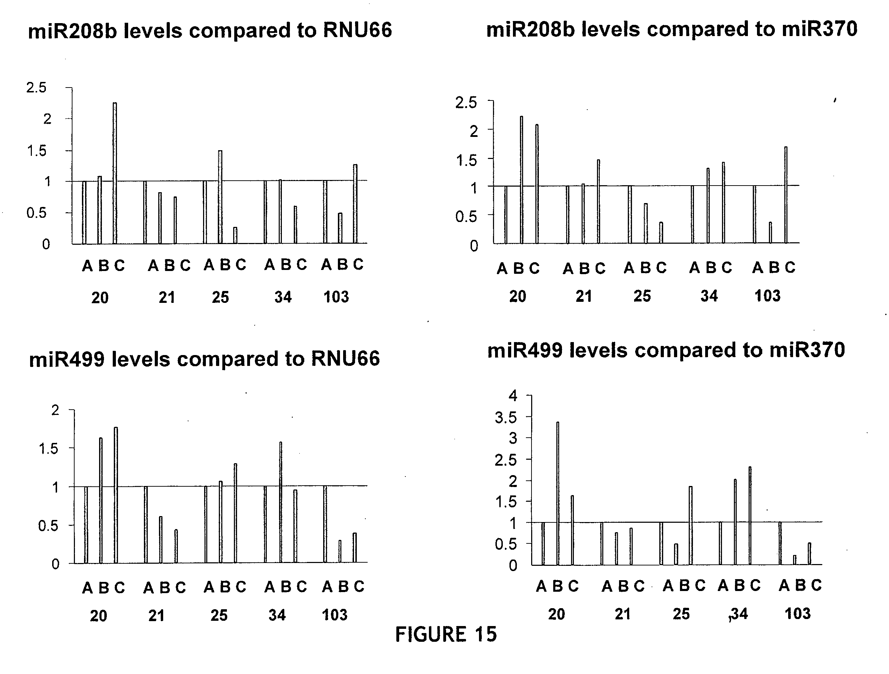

**Figure 15****.** miR-208b and miR-499 expression in human patients treated with β-blockers. RT-PCR analysis was performed on samples from patients treated with β-blockers. Serial biopsies were obtained at end stage heart failure (A), 3 months post-treatment (B) and 12 months post-treatment (C). The expression levels of miR-208b (top panels) and miR-499 (bottom panels) were normalized to the expression of miR-370 (right panels) and the small RNA RNU66 (left panels). Patients 20, 21, 25 and 34 responded to β-blocker treatment, while patient 103 did not respond to the treatment.

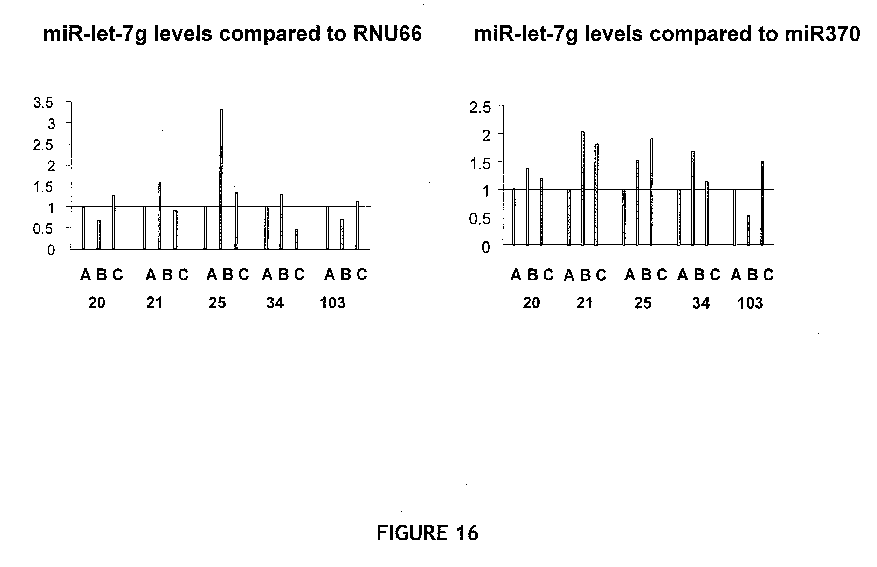

**Figure 16****.** miR-let-7g expression in human patients treated with β-blockers. RT-PCR analysis was performed on samples from patients treated with β-blockers. Serial biopsies were obtained at end stage heart failure (A), 3 months post-treatment (B) and 12 months post-treatment (C). The expression level of miR-let-7g was normalized to the expression of miR-370 (right panel) and the small RNA RNU66 (left panel). Patients 20, 21, 25 and 34 responded to β-blocker treatment, while patient 103 did not respond to the treatment.

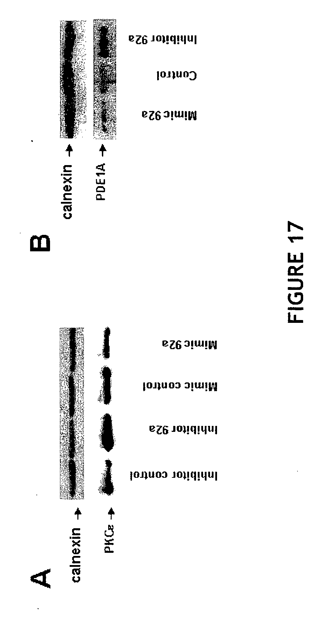

**Figure 17****.** miR-92a targets PKCε and PDE1A. Western blot analysis of rat neonate cardiac myocytes transfected with a miR-92a mimic, a miR-92a inhibitor, or a scrambled control sequence. **A.** Blot probed for PKCε (n=2). **B.** Blot probed for PDE1A (n=1). Calnexin expression was used as a loading control.

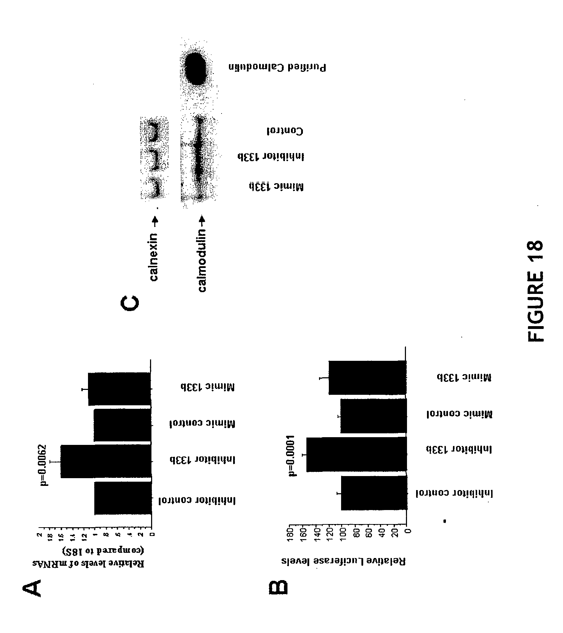

**Figure 18****.** miR-133b targets calmodulin. **A.** RT-PCR analysis for calmodulin mRNA expression in rat neonate cardiac myocytes transfected with a miR-133b mimic, a miR-133b inhibitor, or a scrambled control sequence. Calmodulin expression levels were normalized to those of the 18S ribosomal subunit. **B.** Luciferase activity in rat neonate cardiac myocytes co-transfected with a a construct comprising a luciferase coding region linked to the calmodulin 3'UTR and a miR-133b mimic, a miR-133b inhibitor, or a scrambled control sequence. **C.** Western blot analysis for calmodulin of rat neonate cardiac myocytes transfected with a miR-133b mimic, a miR-133b inhibitor, or a scrambled control sequence. Calnexin expression was used as a loading control. n=3 for all experiments.

### Detailed Description

The present invention is based, in part, on the discovery that specific subsets of microRNAs (miRNAs) are differentially expressed between healthy hearts and failing hearts. Interestingly, some miRNAs are up-regulated or down-regulated in failing hearts of a particular type, such as idiopathic dilated cardiomyopathy or ischemic cardiomyopathy. These differentially expressed miRNAs provide novel biomarkers for heart failure as well as targets for development of effective treatments for heart failure. Accordingly, the present disclosure provides methods of diagnosing or prognosing heart failure in a subject. Methods of treating or preventing heart failure in a subject are also provided.

mRNAs are small, non-coding RNAs of about 18 to about 25 nucleotides, particularly about 21 to about 22 nucleotides in length that are derived from larger precursors. miRNAs act as repressors of target mRNAs by promoting their degradation, when their sequences are perfectly complementary, or by inhibiting translation, when their sequences contain mismatches.

miRNAs are transcribed by RNA polymerase II (pol II) or RNA polymerase III (pol III; see Qi et al. (2006) Cellular & Molecular Immunology Vol. 3:411-419) and can be derived from individual miRNA genes, from introns of protein coding genes, or from poly-cistronic transcripts that often encode multiple, closely related miRNAs. Transcription of miRNA genes by RNA pol II or pol III generate initial transcripts, termed primary miRNA transcripts (pri-miRNAs), that are generally several thousand bases long. In the nucleus, pri-miRNAs are processed by the RNase, Drosha, resulting in 70- to 100-nucleotide hairpin-shaped precursors (pre-miRNAs). Following transport to the cytoplasm, the hairpin pre-miRNA is further processed by Dicer to produce a double stranded miRNA. The mature miRNA strand is then incorporated into the RNA-induced silencing complex (RISC), where it associates with its target mRNAs by base-pair complementarity. In the relatively rare cases in which a miRNA base pairs perfectly with an mRNA target, it promotes mRNA degradation. More commonly, miRNAs form imperfect heteroduplexes with target mRNAs, affecting mRNA translation. The 5' portion of a miRNA spanning bases 2-8, termed the 'seed' region, is especially important for target recognition (Krenz and Robbins (2004) J. Am. Coll. Cardiol., Vol. 44:2390-2397; Kiriazis and Kranias (2000) Annu. Rev. Physiol., Vol. 62:321-351). The sequence of the seed, together with phylogenetic conservation of the target sequence, forms the basis for many current target prediction models.

The present disclosure provides a method of diagnosing or prognosing heart failure in a subject by determining the level of expression of specific miRNAs in a biological sample obtained from the subject. As used herein, the term "heart failure" broadly refers to any condition that reduces the ability of the heart to pump blood. As a result, congestion and edema develop in the tissues. Most frequently, heart failure is caused by decreased contractility of the myocardium, resulting from reduced coronary blood flow; however, many other factors may result in heart failure, including damage to the heart valves, vitamin deficiency, and primary cardiac muscle disease.

In one aspect of the disclosure, the method comprises determining the level of expression of one or more miRNAs in a biological sample from the subject; and comparing the level of expression of one or more miRNAs in said biological sample to the level of one or more miRNAs in a standard sample, wherein a difference in the level of expression in at least one or more miRNAs in said biological sample compared to the level of one or more miRNAs in said standard sample is diagnostic or prognostic for heart failure. The biological sample may include, but is not limited to, blood, serum, plasma, and tissue samples. In a preferred aspect of the disclosure, the biological sample is myocardial tissue (e.g. atrial appendages, endomyocardial biopsies, etc.). A "standard sample" refers to a sample that is representative of a disease-free state, particularly a state in which heart failure or any other associated condition is absent (i.e. a healthy state). By way of example, the standard sample may be a biological sample, such as myocardial tissue, obtained from a healthy subject of similar age as the subject for whom the diagnosis or prognosis is provided. A standard sample may be a composite sample, wherein data obtained from biological samples from several healthy subjects (i.e. control subjects who do not have symptoms of heart failure) are averaged, thereby creating the composite sample.

Methods of determining the level of expression of miRNAs in a sample are known to those skilled in the art. Such methods include polymerase chain reaction (PCR) techniques, such as reverse transcriptase-PCR and real-time PCR, northern blotting, and microarray analysis. In another aspect of the disclosure, the method comprises determining the level of expression of one or more miRNAs selected from the group consisting of hsa-miR-542-5p (SEQ ID NO: 1), hsa-miR-125b (SEQ ID NO: 2), hsa-miR-197 (SEQ ID NO: 3), hsa-miR-195 (SEQ ID NO: 4), hsa-miR-92a (SEQ ID NO: 5), hsa-miR-139 (SEQ ID NO: 6), hsa-miR-100 (SEQ ID NO: 7), hsa-miR-483 (SEQ ID NO: 8), hsa-miR-22 (SEQ ID NO: 9), hsa-miR-23a (SEQ ID NO: 10), hsa-miR-486 (SEQ ID NO: 11), hsa-miR-150 (SEQ ID NO: 12), hsa-miR-30c (SEQ ID NO: 13), hsa-miR-342 (SEQ ID NO: 14), hsa-miR-133a (SEQ ID NO: 15), hsa-miR-422b (also known as miR-378; SEQ ID NO: 16), hsa-miR-221 (SEQ ID NO: 17), hsa-let-7f (SEQ ID NO: 18), hsa-miR-133b (SEQ ID NO: 19), hsa-miR-222 (SEQ ID NO: 20), hsa-miR-224 (SEQ ID NO: 21), hsa-let-7a (SEQ ID NO: 22), hsa-miR-1 (SEQ ID NO: 23), hsa-miR-28 (SEQ ID NO: 24), hsa-miR-199a (SEQ ID NO: 25), hsa-miR-181 b (SEQ ID NO: 26), hsa-miR-20a (SEQ ID NO: 27), hsa-let-7c (SEQ ID NO: 28), hsa-miR-484 (SEQ ID NO: 29), hsa-miR-26b (SEQ ID NO: 30), hsa-let-7d (SEQ ID NO: 31), hsa-miR-10b (SEQ ID NO: 32), hsa-miR-382 (SEQ ID NO: 33), hsa-miR-92b (uauugcacucgucccggccucc; SEQ ID NO: 48), hsa-let-7b (ugagguaguagguugugugguu; SEQ ID NO: 49), hsa-let-7e (ugagguaggagguuguauaguu; SEQ ID NO: 50), and hsa-let-7g (ugagguaguaguuuguacaguu; SEQ ID NO: 51). In another aspect of the disclosure, the one or more miRNAs is selected from hsa-miR-125b (SEQ ID NO: 2), hsa-miR-195 (SEQ ID NO: 4), hsa-miR-92a (SEQ ID NO: 5), hsa-miR-100 (SEQ ID NO: 7), hsa-miR-22 (SEQ ID NO: 9), hsa-miR-486 (SEQ ID NO: 11), hsa-miR-150 (SEQ ID NO: 12), hsa-miR-30c (SEQ ID NO: 13), hsa-miR-342 (SEQ ID NO: 14), hsa-miR-133a (SEQ ID NO: 15), hsa-miR-221 (SEQ ID NO: 17), hsa-let-7f (SEQ ID NO: 18), hsa-miR-133b (SEQ ID NO: 19), hsa-miR-222 (SEQ ID NO: 20), hsa-let-7a (SEQ ID NO: 22), hsa-miR-1 (SEQ ID NO: 23), hsa-miR-28 (SEQ ID NO: 24), and hsa-miR-199a (SEQ ID NO: 25). In yet another aspect of the disclosure, the one or more miRNAs is selected from hsa-miR-195 (SEQ ID NO: 4), hsa-miR-92a (SEQ ID NO: 5), hsa-miR-100 (SEQ ID NO: 7), hsa-miR-486 (SEQ ID NO: 11), hsa-miR-150 (SEQ ID NO: 12), and hsa-miR-422b (also known as miR-378; SEQ ID NO: 16).

In another aspect of the disclosure, the method further comprises diagnosing the type of heart failure based upon the level of one or more miRNAs in said biological sample compared to the level of one or more miRNAs in said standard sample, wherein the level of one or more miRNAs is diagnostic for ischemic cardiomyopathy or idiopathic dilated cardiomyopathy. As used herein, "idiopathic dilated cardiomyopathy" (IDC) is defined as dilation of the left, right, or both ventricles, with impaired contractility of unknown cause. IDC is a common form of dilated cardiomyopathy that produces symptoms of heart failure. Overt congestive heart failure may or may not be present in IDC and arrhythmias are common, and the prognosis is often poor. "Ischemic cardiomyopathy" (ISC), as used herein, refers to patients with a weakness of the muscle in the heart due to inadequate oxygen delivery to the myocardium, often due to coronary artery disease. ISC is a common cause of congestive heart failure and patients with this condition may have at one time experienced a heart attack, angina, or unstable angina. ISC is the most common type of cardiomyopathy in the United States; it affects approximately 1 out of 100 people, most often middle-aged to elderly men.

One or more miRNAs useful for diagnosing IDC or ISC include hsa-miR-125b (SEQ ID NO: 2), hsa-miR-22 (SEQ ID NO: 9), hsa-miR-150 (SEQ ID NO: 12); hsa-miR-30c (SEQ ID NO: 13), hsa-miR-342 (SEQ ID NO: 14), hsa-miR-133a (SEQ ID NO: 15), hsa- miR-422b (also known as miR-378; SEQ ID NO: 16); hsa-let-7f (SEQ ID NO: 18), hsa-miR-133b (SEQ ID NO: 19), hsa-miR-222 (SEQ ID NO: 20), hsa-let-7a (SEQ ID NO: 22), hsa-miR-1 (SEQ ID NO: 23), hsa-miR-28 (SEQ ID NO: 24), and hsa-miR-199a (SEQ ID NO: 25).

The present disclosure also provides a method of treating or preventing heart failure or a disease or condition associated with heart failure in a subject. In one aspect, the method comprises administering a therapeutically effective amount of a miRNA mimic or a miRNA inhibitor to heart cells of the subject. A "therapeutically effective amount" is an amount sufficient to ameliorate or prevent at least one symptom of heart failure. Symptoms of heart failure include, but are not limited to, dyspnea, orthopnea, dizziness, confusion, diaphoresis, peripheral edema, ascites, hepatomegaly, fatigue, nausea, increased heart rate, and pulmonary edema.

In another aspect, the method comprises administering a miRNA mimic or miRNA inhibitor of one or more of the miRNAs selected from the group consisting of: hsa-miR-542-5p (SEQ ID NO: 1), hsa-miR-125b (SEQ ID NO: 2), hsa-miR-197 (SEQ ID NO: 3), hsa-miR-195 (SEQ ID NO: 4), hsa-miR-92a (SEQ ID NO: 5), hsa-miR-139 (SEQ ID NO: 6), hsa-miR-100 (SEQ ID NO: 7), hsa-miR-483 (SEQ ID NO: 8), hsa-miR-22 (SEQ ID NO: 9), hsa-miR-23a (SEQ ID NO: 10), hsa-miR-486 (SEQ ID NO: 11), hsa-miR-150 (SEQ ID NO: 12), hsa-miR-30c (SEQ ID NO: 13), hsa-miR-342 (SEQ ID NO: 14), hsa-miR-133a (SEQ ID NO: 15), hsa-miR-422b (also known as miR-378; SEQ ID NO: 16), hsa-miR-221 (SEQ ID NO: 17), hsa-let-7f (SEQ ID NO: 18), hsa-miR-133b (SEQ ID NO: 19), hsa-miR-222 (SEQ ID NO: 20), hsa-miR-224 (SEQ ID NO:-21), hsa-let-7a (SEQ ID NO: 22), hsa-miR-1 (SEQ ID NO: 23), hsa-miR-28 (SEQ ID NO: 24), hsa-miR-199a (SEQ ID NO: 25), hsa-miR-181 b (SEQ ID NO: 26), hsa-miR-20a (SEQ ID NO: 27), hsa-let-7c (SEQ ID NO: 28), hsa-miR-484 (SEQ ID NO: 29), hsa-miR-26b (SEQ ID NO: 30), hsa-let-7d (SEQ ID NO: 31), hsa-miR-10b (SEQ ID NO: 32), hsa-miR-382 (SEQ ID NO: 33), hsa-miR-92b (uauugcacucgucccggccucc; SEQ ID NO: 48), hsa-let-7b (ugagguaguagguugugugguu; SEQ ID NO: 49), hsa-let-7e (ugagguaggagguuguauaguu; SEQ ID NO: 50), and hsa-let-7g (ugagguaguaguuuguacaguu; SEQ ID NO: 51).

An "miRNA mimic" is an agent used to increase the expression and/or function of a miRNA. The miRNA mimic can also increase, supplement, or replace the function of a natural miRNA. In one aspect, the miRNA mimic may be a polynucleotide comprising the mature miRNA sequence. In another aspect, the miRNA mimic may be a polynucleotide comprising the pri-miRNA or pre-miRNA sequence. The miRNA mimic may contain chemical modifications, such as locked nucleic acids, peptide nucleic acids, sugar modifications, such as 2'-O-alkyl (e.g. 2'-O-methyl, 2'-O-methoxyethyl), 2'-fluoro, and 4' thio modifications, and backbone modifications, such as one or more phosphorothioate, morpholino, or phosphonocarboxylate linkages. Certain miRNA mimics are commercially available from companies, such as Dharmacon (Lafayette, CO) and Ambion, Inc.

In some aspects, the miRNA mimic may be expressed *in vivo* from vectors. A "vector" is a composition of matter which can be used to deliver a nucleic acid of interest to the interior of a cell. Numerous vectors are known in the art including, but not limited to, linear polynucleotides, polynucleotides associated with ionic or amphiphilic compounds, plasmids, and viruses. Thus, the term "vector" includes an autonomously replicating plasmid or a virus. Examples of viral vectors include, but are not limited to, adenoviral vectors, adeno-associated virus vectors, retroviral vectors, and the like. An expression construct can be replicated in a living cell, or it can be made synthetically. For purposes of this application, the terms "expression construct," "expression vector," and "vector," are used interchangeably to demonstrate the application of the invention in a general, illustrative sense, and are not intended to limit the invention.

In one aspect, an expression vector for expressing the miRNA mimic comprises a promoter "operably linked" to a polynucleotide encoding the particular miRNA. The phrase "operably linked" or "under transcriptional control" as used herein means that the promoter is in the correct location and orientation in relation to a polynucleotide to control the initiation of transcription by RNA polymerase and expression of the polynucleotide. The polynucleotide encoding the miRNA may encode the primary-microRNA sequence (pri-miRNA), the precursor-microRNA sequence (pre-miRNA) or the mature miRNA sequence. In a preferred aspect, the polynucleotide comprises the sequence of hsa-miR-133b (SEQ ID NO: 19). The polynucleotide encoding the particular miRNA may be about 18 to about 2000 nucleotides in length, about 70 to about 200 nucleotides in length, about 20 to about 50 nucleotides in length, or about 18 to about 25 nucleotides in length. In other aspects, the polynucleotide encoding the particular miRNA is located in a nucleic acid encoding an intron or in a nucleic acid encoding an untranslated region of an mRNA or in a non-coding RNA.

In some aspects, the miRNA mimic comprises a sequence of hsa-miR-197 (SEQ ID NO: 3), hsa-miR-92a (SEQ ID NO: 5), hsa-miR-139 (SEQ ID NO: 6), hsa-miR-483 (SEQ ID NO: 8), hsa-miR-22 (SEQ ID NO: 9), hsa-miR-486 (SEQ ID NO: 11), hsa-miR-1 50 (SEQ ID NO: 12), hsa-miR-30c (SEQ ID NO: 13), hsa-miR-133a (SEQ ID NO: 15), hsa-miR-422b (also known as miR-378; SEQ ID NO: 16), hsa-miR-221 (SEQ ID NO: 17), hsa-let-7f (SEQ ID NO: 18), hsa-miR-133b (SEQ ID NO: 19), hsa-miR-222 (SEQ ID NO: 20), hsa-miR-224 (SEQ ID NO: 21), hsa-let-7a (SEQ ID NO: 22), hsa-miR-1 (SEQ ID NO: 23), hsa-miR-20a (SEQ ID NO: 27), hsa-let-7c (SEQ ID NO: 28), hsa-miR-484 (SEQ ID NO: 29), hsa-let-7d (SEQ ID NO: 31), or hsa-miR-10b (SEQ ID NO: 32). In a preferred aspect, the miRNA mimic comprises a sequence of hsa-miR-133b (SEQ ID NO: 19).

A "miRNA inhibitor" is an agent that inhibits miRNA function in a sequence-specific manner. In one aspect, the miRNA inhibitor is an antagomir. Initially described by Krützfeldt and colleagues (Krützfeldt et al. (2005) Nature, Vol. 438:685-689), "antagomirs" are single-stranded, chemically-modified ribonucleotides that are at least partially complementary to the miRNA sequence. Antagomirs may comprise one or more modified nucleotides, such as 2'-O-methyl-sugar modifications. In some aspects, antagomirs comprise only modified nucleotides. Antagomirs may also comprise one or more phosphorothioate linkages resulting in a partial or full phosphorothioate backbone. To facilitate *in vivo* delivery and stability, the antagomir may be linked to a cholesterol moiety at its 3' end. Antagomirs suitable for inhibiting miRNAs may be about 15 to about 50 nucleotides in length, more preferably about 18 to about 30 nucleotides in length, and most preferably about 20 to about 25 nucleotides in length. "Partially complementary" refers to a sequence that is at least about 75%, 80%, 85%, 90%, 95%, 96%, 97%, 98%, or 99% complementary to a target polynucleotide sequence. The antagomirs may be at least about 75%, 80%, 85%, 90%, 95%, 96%, 97%, 98%, or 99% complementary to a mature miRNA sequence. In some aspects, the antagomirs are 100% complementary to the mature miRNA sequence.

In another aspect, the miRNA inhibitor is an antisense oligonucleotide targeting the mature miRNA sequence. The antisense oligonucleotides may be comprised of ribonucleotides or deoxyribonucleotides. In some aspects, the antisense oligonucleotides have at least one chemical modification. Antisense oligonucleotides may be comprised of one or more "locked nucleic acids". "Locked nucleic acids" (LNAs) are modified ribonucleotides that contain an extra bridge between the 2' and 4' carbons of the ribose sugar moiety resulting in a "locked" conformation that confers enhanced thermal stability to oligonucleotides containing the LNAs. Alternatively, the antisense oligonucleotides may comprise peptide nucleic acids (PNAs), which contain a peptide-based backbone rather than a sugar-phosphate backbone. Other chemical modifications that the antisense oligonucleotides may contain include, but are not limited to, sugar modifications, such as 2'-O-alkyl (e.g. 2'-O-methyl, 2'-O-methoxyethyl), 2'-fluoro, and 4' thio modifications, and backbone modifications, such as one or more phosphorothioate, morpholino, or phosphonocarboxylate linkages (see, for example, U.S. Patent Nos. 6,693,187 and 7,067,641). In some aspects, suitable antisense oligonucleotides are 2'-O-methoxyethyl "gapmers" which contain 2'-O-methoxyethyl-modified ribonucleotides on both 5' and 3' ends with at least ten deoxyribonucleotides in the center. These "gapmers" are capable of triggering RNase H-dependent degradation mechanisms of RNA targets. Other modifications of antisense oligonucleotides to enhance stability and improve efficacy, such as those described in U.S. Patent No. 6,838,283, are known in the art and are suitable for use in the methods of the disclosure. Preferable antisense oligonucleotides useful for inhibiting the activity of miRNAs are about 19 to about 25 nucleotides in length. Antisense oligonucleotides may comprise a sequence that is at least partially complementary to a mature miRNA sequence, e.g. at least about 75%, 80%, 85%, 90%, 95%, 96%, 97%, 98%, or 99% complementary to a mature miRNA sequence. In one aspect, the antisense oligonucleotide comprises a sequence that is 100% complementary to a mature miRNA sequence.

In some aspects of the disclosure, the miRNA inhibitor comprises a sequence complementary to hsa-miR-125b (SEQ ID NO: 2), hsa-miR-195 (SEQ ID NO: 4), hsa-miR-100 (SEQ ID NO: 7), hsa-miR-23a (SEQ ID NO: 10), hsa-miR-342 (SEQ ID NO: 14), hsa-miR-28 (SEQ ID NO: 24), hsa-miR-199a (SEQ ID NO: 25), hsa-miR-181b (SEQ ID NO: 26), hsa-miR-26b (SEQ ID NO: 30), or hsa-miR-382 (SEQ ID NO: 33). In a preferred aspect, the miRNA inhibitor comprises a sequence complementary to hsa-miR-100 (SEQ ID NO: 7).

In yet another aspect, the miRNA inhibitor is an inhibitory RNA molecule having at least partial sequence identity to the mature miRNA sequence. The inhibitory RNA molecule may be a double-stranded, small interfering RNA (siRNA) or a short hairpin RNA molecule (shRNA) comprising a stem-loop structure. The double-stranded regions of the inhibitory RNA molecule may comprise a sequence having at least about 75%, 80%, 85%, 90%, 95%, 96%, 97%, 98%, or 99% identity to the mature miRNA sequence. In some aspects, the double-stranded regions of the inhibitory RNA molecule may contain 100% identity to the target miRNA sequence. The inhibitory RNA molecule may comprise a sequence having at least partial sequence identity to hsa-miR-125b (SEQ ID NO: 2), hsa-miR-195 (SEQ ID NO: 4), hsa-miR-100 (SEQ ID NO: 7), hsa-miR-23a (SEQ ID NO: 10), hsa-miR-342 (SEQ ID NO: 14), hsa-miR-28 (SEQ ID NO: 24), hsa-miR-199a (SEQ ID NO: 25), hsa-miR-181b (SEQ ID NO: 26), hsa-miR-26b (SEQ ID NO: 30), or hsa-miR-382 (SEQ ID NO: 33).

The miRNA inhibitor may be produced *in vivo* from an expression vector. In one aspect, an expression vector for expressing a miRNA inhibitor comprises a promoter operably linked to a polynucleotide encoding an antisense oligonucleotide, wherein the sequence of the expressed antisense oligonucleotide is complementary to the mature miRNA sequence. In another aspect, an expression vector for expressing a miRNA inhibitor comprises one or more promoters operably linked to a polynucleotide encoding a shRNA or siRNA, wherein the expressed shRNA or siRNA comprises a sequence that is identical or substantially identical to the mature miRNA sequence. "Substantially identical" refers to a sequence that is at least about 75%, 80%, 85%, 90%, 95%, 96%, 97%, 98%, or 99% identical to a target polynucleotide sequence.

In another aspect of the disclosure, the method of treating or preventing heart failure or a disease or condition associated with heart failure in a subject further comprises administering to the subject a second cardiac therapy. A cardiac therapy encompasses any therapy that is typically used to treat one or more symptoms of heart failure or a disease or condition associated with heart failure. The second cardiac therapy may include, without limitation, so-called "beta blockers," anti-hypertensives, cardiotonics, anti-thrombotics, vasodilators, hormone antagonists, iontropes, diuretics, endothelin receptor antagonists, calcium channel blockers, phosphodiesterase inhibitors, ACE inhibitors, angiotensin type 2 antagonists and cytokine blockers/inhibitors, HDAC inhibitors, and histone acetylase transferase (HAT) modulators. In one aspect of the disclosure, the second cardiac therapy is an inotropic agent, a neurohumoral blocker, an aldosterone antagonist, a diuretic, a vasodilator, an endothelin receptor antagonist, or a HDAC inibitor.

An "inotropic agent" refers to an agent which increases or decreases force or energy of muscular contractions, especially drugs that increase the strength of myocardial contractility. Positive inotropic agents may include calcium; the calcium sensitizer levosimendan; the cardiac glycoside, digoxin; catecholamines, such as dopamine, dobutamine, dopexamine, epinephrine, isoproterenol, and norepinephrine; and phosphodiesterase inhibitors, such as enoximone, milrinone, and theophylline. Negative inotropic agents include beta adrenergic blockers, such as labetalol and carvedilol; and calcium channel blockers, such as diltiazam and verapamil.

A "neurohumoral blocker" refers to a drug which blocks a neurohumoral response and includes beta adrenergic blockers, such as bisoprolol, carvedilol, and metoprolol; and angiotensin converting enzyme inhibitors, such as enalapril, lisinopril, ramipril, and captopril.

A "diuretic" refers to a drug prescribed for fluid retention and swelling of feet, legs and abdomen. Diuretics prompt the kidneys to filter more sodium and water from the blood. With less fluid in the body, the heart can pump and circulate blood with less effort. Additionally, diuretics can decrease fluid retention in the lungs, ankles, legs and other parts of the body. Diuretics include, for example, furosemide, bumetanide, metolazone, spironolactone, and eplerenone.

An "aldosterone antagonist" refers to a drug which antagonizes the action of aldosterone at mineralocorticoid receptors. This group of drugs is often used as adjunctive therapy, in combination with other drugs, for the management of chronic heart failure. Aldosterone antagonists include, for example, spironolactone and eplerenone.

A "vasodilator" refers to a drug that dilates, or causes an increase in the diameter of blood vessels. Vasodilators include, for example, hydralazine, isosorbide dinitrate and isosorbide mononitrate, captopril and longer lasting agents such as metamine, paveril, nitroglyn and peritrate.

Endothelin (ET) is a 21-amino acid peptide that has potent physiologic and pathophysiologic effects that appear to be involved in the development of heart failure. The effects of ET are mediated through interaction with two classes of cell surface receptors. The type A receptor (ET-A) is associated with vasoconstriction and cell growth while the type B receptor (ET-B) is associated with endothelial-cell mediated vasodilation and with the release of other neurohormones, such as aldosterone. Pharmacologic agents that can inhibit either the production of ET or its ability to stimulate relevant cells are known in the art. Inhibiting the production of ET involves the use of agents that block an enzyme termed endothelin-converting enzyme that is involved in the processing of the active peptide from its precursor. Inhibiting the ability of ET to stimulate cells involves the use of agents that block the interaction of ET with its receptors. Non-limiting examples of endothelin receptor antagonists (ERA) include Bosentan, Enrasentan, Ambrisentan, Darusentan, Tezosentan, Atrasentan, Avosentan, Clazosentan, Edonentan, sitaxsentan, TBC 3711, BQ 123, and BQ 788.

The second cardiac therapy may be co-administered (e.g. simultaenously) to the subject with the miRNA mimic or miRNA inhibitor. Alternatively, the second cardiac therapy may be administered before or after the miRNA mimic or miRNA inhibitor. The time interval between administration of the miRNA mimic or miRNA inhibitor and administration of the second cardiac therapy may range from minutes to weeks. In some cases, the two therapies may be administered within 12-24 hours of each other, more preferably within about 6-12 hours of each other, and most preferably within about 12 hours of each other. In some situations, it may be desirable to extend the time period for treatment significantly, however, where several days (2, 3, 4, 5, 6 or 7) to several weeks (1, 2, 3, 4, 5, 6, 7 or 8) lapse between the respective administrations.

The present disclosure also encompasses a method of treating or preventing heart failure or a disease or condition associated with heart failure in a subject comprising administering to the subject a pharmaceutical composition, wherein said pharmaceutical composition comprises at least one miRNA mimic or at least one miRNA inhibitor and a pharmaceutically acceptable carrier. In one aspect, the at least one miRNA mimic or at least one miRNA inhibitor is a mimic or inhibitor of one or more of the miRNAs selected from the group consisting of hsa-miR-542-5p (SEQ ID NO: 1), hsa-miR-125b (SEQ ID NO: 2), hsa-miR-197 (SEQ ID NO: 3), hsa-miR-195 (SEQ ID NO: 4), hsa-miR-· 92a (SEQ ID NO: 5), hsa-miR-139 (SEQ ID NO: 6), hsa-miR-100 (SEQ ID NO: 7), hsa-miR-483 (SEQ ID NO: 8), hsa-miR-22 (SEQ ID NO: 9), hsa-miR-23a (SEQ ID NO: 10), hsa-miR-486 (SEQ ID NO: 11), hsa-miR150 (SEQ ID NO: 12), hsa-miR-30c (SEQ ID NO: 13), hsa-miR-342 (SEQ ID NO: 14), hsa-miR-133a (SEQ ID NO: 15), hsa-miR-422b (also known as miR-378; SEQ ID NO: 16), hsa-miR-221 (SEQ ID NO: 17), hsa-let-7f (SEQ ID NO: 18), hsa-miR-133b (SEQ ID NO: 19), hsa-miR-222 (SEQ ID NO: 20), hsa-miR-224 (SEQ ID NO: 21), hsa-let-7a (SEQ ID NO: 22), hsa-miR-1 (SEQ ID NO: 23), hsa-miR-28 (SEQ ID NO: 24), hsa-miR-199a (SEQ ID NO: 25), hsa-miR-181b (SEQ ID NO: 26), hsa-miR-20a (SEQ ID NO: 27), hsa-let-7c (SEQ ID NO: 28), hsa-miR-484 (SEQ ID NO: 29), hsa-miR-26b (SEQ ID NO: 30), hsa-let-7d (SEQ ID NO: 31), hsa-miR-10b (SEQ ID NO: 32;), hsa-miR-382 (SEQ ID NO: 33), hsa-miR-92b (uauugcacucgucccggccucc; SEQ ID NO: 48), hsa-let-7b (ugagguaguagguugugugguu; SEQ ID NO: 49), hsa-let-7e (ugagguaggagguuguauaguu; SEQ ID NO: 50), and hsa-let-7g (ugagguaguaguuuguacaguu; SEQ ID NO: 51). The pharmaceutical composition may comprise a second cardiac therapy that is typically prescribed to treat heart failure as described above. In one aspect, the pharmaceutical composition further comprises one or more of an inotropic agent, a neurohumoral blocker, an aldosterone antagonist, a diuretic, a vasodilator, an endothelin receptor antagonist, or a HDAC inibitor.

In another aspect, the pharmaceutical composition comprises at least one miRNA mimic, wherein said miRNA mimic comprises a sequence of hsa-miR-197 (SEQ ID NO: 3), hsa-miR-92a (SEQ ID NO: 5), hsa-miR-139 (SEQ ID NO: 6), hsa-miR-483 (SEQ ID NO: 8), hsa-miR-22 (SEQ ID NO: 9), hsa-miR-486 (SEQ ID NO: 11), hsa-miR-150 (SEQ ID NO: 12), hsa-miR-30c (SEQ ID NO: 13), hsa-miR-133a (SEQ ID NO: 15), hsa-miR-422b (also known as miR-378; SEQ ID NO: 16), hsa-miR-221 (SEQ ID NO: 17), hsa-let-7f (SEQ ID NO: 18), hsa-miR-133b (SEQ ID NO: 19), hsa-miR-222 (SEQ ID NO: 20), hsa-miR-224 (SEQ ID NO: 21), hsa-let-7a (SEQ ID NO: 22), hsa-miR-1 (SEQ ID NO: 23), hsa-miR-20a (SEQ ID NO: 27), hsa-let-7c (SEQ ID NO: 28), hsa-miR-484 (SEQ ID NO: 29), hsa-let-7d (SEQ ID NO: 31), or hsa-miR-10b (SEQ ID NO: 32). In still another aspect, the pharmaceutical composition comprises at least one miRNA inhibitor, wherein said miRNA inhibitor comprises a sequence complementary to hsa-miR-125b (SEQ ID NO: 2), hsa-miR-195 (SEQ ID NO: 4), hsa-miR-100 (SEQ ID NO: 7), hsa-miR-23a (SEQ ID NO: 10), hsa-miR-342 (SEQ ID NO: 14), hsa-miR-28 (SEQ ID NO: 24), hsa-miR-199a (SEQ ID NO: 25), hsa-miR-181b (SEQ ID NO: 26), hsa-miR-26b (SEQ ID NO: 30), or hsa-miR-382 (SEQ ID NO: 33).

The pharmaceutical compositions can be administered for any of the uses described herein by any suitable means, for example, orally, such as in the form of tablets, capsules, granules or powders; sublingually; bucally; parenterally, such as by subcutaneous, intravenous, intramuscular, or intracisternal injection or infusion techniques (e.g., as sterile injectable aqueous or non-aqueous solutions or suspensions); nasally, including administration to the nasal membranes, such as by inhalation spray; topically, such as in the form of a cream or ointment; or rectally such as in the form of suppositories; in dosage unit formulations containing non-toxic, pharmaceutically acceptable carriers or diluents. As used herein, "pharmaceutically acceptable carrier" includes solvents, buffers, solutions, dispersion media, coatings, antibacterial and antifungal agents, isotonic and absorption delaying agents and the like acceptable for use in formulating pharmaceuticals, such as pharmaceuticals suitable for administration to humans.

Pharmaceutical compositions comprising miRNA inhibitors or miRNA mimics may also be administered by catheter systems or systems that isolate coronary circulation for delivering therapeutic agents to the heart. Various catheter systems for delivering therapeutic agents to the heart and coronary vasculature are known in the art. Some non-limiting examples of catheter-based delivery methods or coronary isolation methods suitable for use are disclosed in U.S. Patent No. 6,416,510; U.S. Patent No. 6,716,196; U.S. Patent No. 6,953,466, WO 2005/082440, WO 2006/089340, U.S. Patent Publication No. 2007/0203445, U.S. Patent Publication No. 2006/0148742, and U.S. Patent Publication No. 2007/0060907. The miRNA mimics or miRNA inhibitors can, for example, be administered in a form suitable for immediate release or extended release. Immediate release or extended release can be achieved by the use of suitable pharmaceutical compositions comprising the miRNA mimics or miRNA inhibitors, or, particularly in the case of extended release, by the use of devices such as subcutaneous implants or osmotic pumps.

Colloidal dispersion systems, such as macromolecule complexes, nanocapsules, microspheres, beads, and lipid-based systems including oil-in-water emulsions, micelles, mixed micelles, and liposomes, may be used as delivery vehicles for the miRNA mimics or miRNA inhibitors. Commercial available fat emulsions that are suitable for delivering the nucleic acids of the disclosure to cardiac and skeletal muscle tissues include Intralipid^{®}, Liposyn^{®}, Liposyn^{®} II, Liposyn^{®} III, Nutrilipid, and other similar lipid emulsions. A preferred colloidal system for use as a delivery vehicle *in vivo* is a liposome (i.e., an artificial membrane vesicle). The preparation and use of such systems is well known in the art. Exemplary formulations are also disclosed in US 5,981,505; US 6,217,900; 6,383,512; US 5,783,565; US 7,202,227; US 6,379,965; US 6,127,170; US 5,837,533; US 6,747,014; and WO03/093449.

Exemplary compositions for oral administration include suspensions which can contain, for example, microcrystalline cellulose for imparting bulk, alginic acid or sodium alginate as a suspending agent, methylcellulose as a viscosity enhancer, and sweeteners or flavoring agents such as those known in the art; and immediate release tablets which can contain, for example, microcrystalline cellulose, dicalcium phosphate, starch, magnesium stearate and/or lactose and/or other excipients, binders, extenders, disintegrants, diluents and lubricants such as those known in the art. The miRNA mimics and miRNA inhibitors can also be delivered through the oral cavity by sublingual and/or buccal administration. Molded tablets, compressed tablets or freeze-dried tablets are exemplary forms which may be used. Exemplary compositions include those formulating the present compound(s) with fast dissolving diluents such as mannitol, lactose, sucrose and/or cyclodextrins. Also included in such formulations may be high molecular weight excipients such as celluloses (avicel) or polyethylene glycols (PEG). Such formulations can also include an excipient to aid mucosal adhesion such as hydroxy propyl cellulose (HPC), hydroxy propyl methyl cellulose (HPMC), sodium carboxy methyl cellulose (SCMC), maleic anhydride copolymer (e.g., Gantrez), and agents to control release such as polyacrylic copolymer (e.g. Carbopol 934). Lubricants, glidants, flavors, coloring agents and stabilizers may also be added for ease of fabrication and use.

Exemplary compositions for nasal aerosol or inhalation administration include solutions in saline which can contain, for example, benzyl alcohol or other suitable preservatives, absorption promoters to enhance bioavailability, and/or other solubilizing or dispersing agents such as those known in the art.

Exemplary compositions for parenteral administration include injectable solutions or suspensions which can contain, for example, suitable non-toxic, parenterally acceptable diluents or solvents, such as mannitol, 1,3-butanediol, water, Ringer's solution, an isotonic sodium chloride solution, or other suitable dispersing or wetting and suspending agents, including synthetic mono- or diglycerides, and fatty acids, including oleic acid, or Cremaphor.

Exemplary compositions for rectal administration include suppositories which can contain, for example, a suitable non-irritating excipient, such as cocoa butter, synthetic glyceride esters or polyethylene glycols, which are solid at ordinary temperatures, but liquify and/or dissolve in the rectal cavity to release the drug. Exemplary compositions for topical administration include a topical carrier such as Plastibase (mineral oil gelled with polyethylene).

It will be understood that the specific dose level and frequency of dosage for any particular subject can be varied and will depend upon a variety of factors including the activity of the specific compound employed (e.g. specific miRNA mimic or miRNA inhibitor), the metabolic stability and length of action of that compound, the species, age, body weight, general health, sex and diet of the subject, the mode and time of administration, rate of excretion, drug combination, and severity of the particular condition.

The present disclosure also contemplates a method of regulating reactivation of the fetal cardiac gene program (FGP) in a cell. In one aspect, the method comprises providing a cardiac cell with one or more miRNA inhibitors or miRNA mimics, wherein the one or more miRNA inhibitors or miRNA mimics modulates expression of at least one marker of the fetal cardiac gene program. The term "cardiac cell" refers to cardiac myocytes and/or cardiac endothelial cells. According to certain aspects of the disclosure the term includes cardiac fibroblasts and/or other cell types present in the heart such as smooth muscle cells (e.g., in the walls of cardiac blood vessels), neurons and glial cells in cardiac nerves, etc. Reactivation of the fetal cardiac gene program (FGP) is a characteristic feature of hypertrophied and failing hearts that correlates with impaired cardiac function and poor prognosis. ( Kuwahara et al., 2003, EMBO J. 22 (23): 6310-6321) Beta-myosin heavy chain (βMyHC), alpha-myosin heavy chain (αMyHC), atrial natriuretic factor (ANF), brain natriuretic peptide (BNP), SERCA2a, and alpha-skeletal actin (Sk α-actin) are among the marker genes that reflect activation of the FGP and whose expression is altered in heart failure, and may adversely impact on disease progression. In some aspects, the expression of at least one marker of the fetal cardiac program is modulated, wherein the at least one marker of the fetal cardiac gene program is selected from the group consisting of βMyHC, αMyHC, ANF, BNP, SERCA2a, and skeletal α-actin.

Suitable miRNA inhibitors or miRNA mimics for use in regulating the reactivation of the FGP include one or more miRNA inhibitors or miRNA mimics of a miRNA selected from the group consisting of hsa-miR-542-5p (SEQ ID NO: 1), hsa-miR-125b (SEQ ID NO: 2), hsa-miR-197 (SEQ ID NO: 3), hsa-miR-195 (SEQ ID NO: 4), hsa-miR-92a (SEQ ID NO: 5), hsa-miR-139 (SEQ ID NO: 6), hsa-miR-100 (SEQ ID NO: 7), hsa-miR-483 (SEQ ID NO: 8), hsa-miR-22 (SEQ ID NO: 9), hsa-miR-23a (SEQ ID NO: 10), hsa-miR-486 (SEQ ID NO: 11), hsa-miR150 (SEQ ID NO: 12), hsa-miR-30c (SEQ ID NO: 13), hsa-miR-342 (SEQ ID NO: 14), hsa-miR-133a (SEQ ID NO: 15), hsa-miR-422b (also known as miR-378; SEQ ID NO: 16), hsa-miR-221 (SEQ ID NO: 17), hsa-let-7f (SEQ ID NO: 18), hsa-miR-133b (SEQ ID NO: 19), hsa-miR-222 (SEQ ID NO: 20), hsa-miR-224 (SEQ ID NO: 21), hsa-let-7a (SEQ ID NO: 22), hsa-miR-1 (SEQ ID NO: 23), hsa-miR-28 (SEQ ID NO: 24), hsa-miR-199a (SEQ ID NO: 25), hsa-miR-181b (SEQ ID NO: 26), hsa-miR-20a (SEQ ID NO: 27), hsa-let-7c (SEQ ID NO: 28), hsa-miR-484 (SEQ ID NO: 29), hsa-miR-26b (SEQ ID NO: 30), hsa-let-7d (SEQ ID NO: 31), hsa-miR-10b (SEQ ID NO: 32), hsa-miR-382 (SEQ ID NO: 33), hsa-miR-92b (uauugcacucgucccggccucc; SEQ ID NO: 48), hsa-let-7b (ugagguaguagguugugugguu; SEQ ID NO: 49), hsa-let-7e (ugagguaggagguuguauaguu; SEQ ID NO: 50), and hsa-let-7g (ugagguaguaguuuguacaguu; SEQ ID NO: 51). In one aspect, the one or more miRNA inhibitors or miRNA mimics is a mimic or inhibitor of hsa-miR-92a (SEQ ID NO: 5), hsa-miR-100 (SEQ ID NO: 7), or hsa-miR-133b (SEQ ID NO: 19).

The present disclosure also contemplates a method of monitoring treatment for heart failure in a subject. In one aspect, the method comprises obtaining a first biological sample from the subject at a first time point, wherein the first time point is prior to the start of a drug therapy protocol for heart failure; obtaining a second biological sample from the subject at a second time point, wherein the second time point is after the start of a drug therapy protocol for heart failure; processing the first biological sample and second biological sample by miRNA array analysis, mRNA array analysis and targeted proteomic expression analysis; and comparing miRNA, mRNA, and protein expression data from the first biological sample to the expression data from the second biological sample to determine changes in gene expression patterns, wherein changes in gene expression are indicative of the efficacy of the drug therapy protocol. The first time point and the second time point may be about three to about eighteen months apart, about three to about twelve months apart, or preferably about three to about nine months apart. Additional biological samples may be obtained from each patient at several time points after the initiation of the drug therapy protocol. In some cases, two or more samples are collected after the start of the drug therapy protocol. In other cases, three or more samples are collected after the start of the drug therapy protocol. The samples may be collected about every three months, about every six months, about every nine months, or about every tweleve months.

The drug therapy protocol used to treat the subject may be any standard drug therapy protocol to treat heart failure, such as ACE-inhibitors (e.g. captopril, lisinopril, etc.), beta blockers (e.g. metoprolol, carvedilol), aldosterone antagonists, diuretics (e.g. furosemide, spirolactone, eplerenone), and digoxin. The drug therapy protocol may also be any of the treatment methods comprising administering one or more miRNA mimics or miRNA inhibitors disclosed herein. The dosage or type of drug therapy may be adjusted based on the expression profile data obtained at one or more time points posttreatment. The method allows for the tailoring of a particular drug therapy regimen for treating heart failure in a particular patient.

This invention is further illustrated by the following additional examples that should not be construed as limiting.

### Examples

### Example 1. miRNAs are differentially expressed between failing and non-failing human hearts

Using array-based technologies, expression levels of cardiac derived miRNAs were compared in human heart tissues from non-failing (NF) and failing human hearts. Failing hearts included those resulting from idiopathic dilated cardiomyopathy (IDC) and ischemic cardiomyopathy (ISC). Bioinformatic analysis of 470 microRNAs (Sanger miRBase Release 9.0), was used to identify a number of differentially expressed miRNAs in non-failing versus failing hearts. A unique "fingerprint" of miRNA expression was revealed with specific miRNAs being either increased or decreased in expression. These miRNAs represent biomarkers of heart failure, as well as novel therapeutic targets in the treatment of heart failure.

In a comparison of miRNA expression between NF and IDC hearts, changes in expression were noted for 22 miRNAs, 6 of which increased in IDC compared to NF hearts (hsa-miR-125b, hsa-miR-195, hsa-miR-100, hsa-miR-181b, hsa-miR-23a, and hsa-miR-382) and 16 of which were decreased in IDC compared to NF hearts (hsa-miR-133a, hsa-miR-133b, hsa-miR-221, hsa-miR-197, hsa-miR-30c, hsa-miR-92, hsa-miR-22, hsa-miR-486, hsa-miR-542-5p, hsa-miR-139, hsa-miR-150, hsa-miR-10b, hsa-miR-483, hsa-miR-594, hsa-miR-422b (also known as miR-378), and hsa-miR-20a) as shown in Figure 1A and Figure 2. Sequences of mature miRNAs of interest are shown in Table 1 (below), based upon Sanger miRBase ver. 9.2.

In a comparison of miRNA expression between NF and ISC hearts, changes in expression were noted for 22 miRNAs, 6 of which increased in ISC compared to NF hearts (hsa-miR-195, hsa-miR-100, hsa-miR-342, hsa-miR-28, hsa-miR-199a, and hsa-miR-26b) and 16 of which were decreased in ISC (hsa-miR-92, hsa-miR-221, hsa-miR-486, hsa-miR-133a, hsa-miR-150, hsa-miR-422b (also known as miR-378), let-7f, let-7a, hsa-miR-1, hsa-miR-222, let-7c, hsa-miR-133b, hsa-miR-224, hsa-miR-484, hsa-miR-594, and let-7d) compared to NF hearts as shown in Figure 1B and Figure 2. Sequences of selected mature miRNAs are shown in Table 1 (below), based upon Sanger miRBase ver. 9.2.

| **Table 1. miRNA Sequences** | | | |
|---|---|---|---|
| Sanger ID | Accession | Mature Sequence (5' to 3') | Number |
| hsa-miR-542-5p | MIMAT0003340 | UCGGGGAUCAUCAUGUCACGAG | SEQ ID NO: 1 |
| hsa-miR-125b | MIMAT0000423 | UCCCUGAGACCCUAACUUGUGA | SEQ ID NO: 2 |
| hsa-miR-197 | MIMAT0000227 | UUCACCACCUUCUCCACCCAGC | SEQ ID NO: 3 |
| hsa-miR-195 | MIMAT0000461 | UAGCAGCACAGAAAUAUUGGC | SEQ ID NO: 4 |
| hsa-miR-92a‡ | M1MAT0000092 | UAUUGCACUUGUCCCCGGCCUGU | SEQ ID NO: 5 |
| hsa-miR-139 | MIMAT0000250 | UCUACAGUGCACGUGUCU | SEQ ID NO: 6 |
| hsa-miR-100 | MIMAT0000098 | AACCCGUAGAUCCGAACUUGUG | SEQ ID NO: 7 |
| hsa-miR-483 | MIMAT0002173 | UCACUCCUCUCCUCCCGUCUUCU | SEQ ID NO: 8 |
| hsa-miR-22 | MIMAT0000077 | AAGCUGCCAGUUGAAGAACUGU | SEQ ID NO: 9 |
| hsa-miR23a | MIMAT0000078 | AUCACAUUGCCAGGGAUUUCC | SEQ ID NO: 10 |
| hsa-miR486 | MIMAT0002177 | UCCUGUACUGAGCUGCCCCGAG | SEQ ID NO: 11 |
| hsa-miR150 | MIMAT000451 | UCUCCCAACCCUUGUACCAGUG | SEQ ID NO: 12 |
| hsa-miR-30c | MIMAT0000244 | UGUAAACAUCCUACACUCUCAGC | SEQ ID NO: 13 |
| hsa-miR-342 | MIMAT0000753 | UCUCACACAGAAAUCGCACCCGUC | SEQ ID NO: 14 |
| hsa-miR-133a | MIMAT0000427 | UUGGUCCCCUUCAACCAGCUGU | SEQ ID NO: 15 |
| hsa-miR-422b† | MIMAT0000732 | CUGGACUUGGAGUCAGAAGGCC | SEQ ID NO: 16 |
| hsa-miR-221 | MIMAT0000278 | AGCUACAUUGUCUGCUGGGUUUC | SEQ ID NO: 17 |
| hsa-let-7f | MIMAT0000067 | UGAGGUAGUAGAUUGUAUAGUU | SEQ ID NO: 18 |
| hsa-miR-133b | MIMAT0000770 | UUGGUCCCCUUCAACCAGCUA | SEQ ID NO: 19 |
| hsa-miR-222 | MIMAT0000279 | AGCUACAUCUGGCUACUGGGUCUC | SEQ ID NO: 20 |
| hsa-miR-224 | MIMAT0000281 | CAAGUCACUAGUGGUUCCGUUUA | SEQ ID NO: 21 |
| hsa-let-7a | MIMAT0000062 | UGAGGUAGUAGGUUGUAUAGUU | SEQ ID NO: 22 |
| hsa-miR-1 | MIMAT0000416 | UGGAAUGUAAAGAAGUAUGUA | SEQ ID NO: 23 |
| hsa-miR-28 | MIMAT0000085 | AAGGAGCUCACAGUCUAUUGAG | SEQ ID NO: 24 |
| hsa-miR-199a | MIMAT0000231 | CCCAGUGUUCAGACUACCUGUUC | SEQ ID NO: 25 |
| hsa-miR-181b | MIMAT0000257 | AACAUUCAUUGCUGUCGGUGGG | SEQ ID NO: 26 |
| hsa-miR-20a | MIMAT0000075 | UAAAGUGCUUAUAGUGCAGGUAG | SEQ ID NO: 27 |
| hsa-let-7c | MIMAT0000064 | UGAGGUAGUAGGUUGUAUGGUU | SEQ ID NO: 28 |
| hsa-miR-484 | MIMAT0002174 | UCAGGCUCAGUCCCCUCCCGAU | SEQ ID NO: 29 |
| hsa-miR-26b | MIMAT0000083 | UUCAAGUAAUUCAGGAUAGGUU | SEQ ID NO: 30 |
| hsa-let-7d | MIMAT0000065 | AGAGGUAGUAGGUUGCAUAGU | SEQ ID NO: 31 |
| hsa miR-594** | MI0003606 | | |
| hsa-miR-10b | MIMAT0000254 | UACCCUGUAGAACCGAAUUUGU | SEQ ID NO: 32 |
| hsa-miR-382 | MIMAT0000737 | GAAGUUGUUCGUGGUGGAUUCG | SEQ ID NO: 33 |

| | | | |
|---|---|---|---|
| **hsa-miR-594 is a fragment of an annotated tRNA gene in miRBase. † hsa-miR-422b has been re-named miR-378 in the Sanger miRBase database (Release 11.0) ‡ has-miR-92 has been re-named miR-92a in the Sanger miRBase database (Release 11.0) | | | |

Figure 3 shows comparison of human heart miR expression data, with p < 0.05, (unpaired t-test), for NF vs. IDC and NF vs. ISC. Expression of three miRNAs were significantly increased in IDC over NF hearts (hsa-miR-125b, hsa-miR-195 and hsa-miR-100), while expression of six miRNAs were significantly decreased in IDC compared to NF hearts (hsa-miR-22, hsa-miR-92, hsa-miR-221, hsa-miR-486, hsa-miR-30c, and hsa-miR-133a). Expression of five miRNAs were significantly increased in ISC over NF hearts (hsa-miR-195, hsa-miR-100, hsa-miR-342, hsa-miR-28, and hsa-miR-199a), while expression of ten miRNAs were significantly decreased in ISC compared to NF hearts (hsa-miR-92, hsa-miR-221, hsa-miR-486, hsa-miR-133a, hsa-miR-150, hsa-miR-422b (also known as miR-378), let-7f, let-7a, hsa-miR-1, and hsa-miR-222).

The results of a composite analysis comparing miRNA expression levels between NF vs. IDC hearts and NF vs. ISC hearts demonstrated concordant regulation of several miRNAs as well as miRNAs that are regulated in a unique, heart failure sub-type specific manner (Figure 2). miRNAs hsa-miR-92, hsa-miR-221, hsa-miR-486, hsa-miR-150 and hsa-miR-422b were significantly down-regulated in both IDC and ISC hearts, while hsa-miR-195 and hsa-miR-100 were up-regulated in both IDC and ISC hearts. The latter result is in agreement with Rooij *et al.,* 2006, who previously demonstrated that miR-195 is up-regulated in failing (IDC) human hearts, as well as in transgenic mice with mechanically- or genetically-induced pathological hypertrophy.

A detailed analysis of the relative expression of each of the detectable miRNAs from the Sanger human miRBase 9.0 data set was derived from the complete data set. All hearts had between 87 and 238 (out of a possible 454) quantifiably detectable miR transcripts (data not show).

### Specific Methods

Micro RNA extraction: miRNA was extracted from the left ventricle of human hearts from 6 non-failing (NF), 5 idiopathic dilated cardiomyopathy (IDC) and 5 ischemic cardiomyopathy (ISC) patients. All patients were males between 50-65 years old. At the time of cardiac explantation, patients were receiving a number of pharmacological agents including ACE inhibitors, diuretics, beta-blockers, digitalis, anticoagulants, insulin, beta-agonists, and nitrates. The cardiac performance of each of the patients was assessed at the time of cardiac explantation by echocardiography. The percentage of ejection fraction for each of the groups were as follows: NF: 64 ± 4.3%; IDC: 12 ± 3.1%; and ISC: 18.6 ± 3.5 (mean ± SEM). Extraction was performed using the mirVana™ kit (Ambion) according to manufacturer's recommendation.

Array analysis: Samples were submitted to LC Sciences, LLC, Houston, Texas, for microRNA Detection Microarray Service. Bioinformatic analysis was used to identify relative expression of 470 microRNAs (Sanger miRBase Release 9.0). This analysis identified a number of differentially expressed miRNAs in nonfailing (NF) versus failing hearts (ISC and/or IDC). Two categories of failing hearts were examined, those derived from patients with idiopathic dilated cardiomyopathy (IDC) or those with ischemic cardiomyopathy (ISC). In the NF group, six hearts (n = 6) were examined. Comparing NF to IDC hearts (n = 5), statistically significant changes (p < 0.05, unpaired t-test) were noted for 12 miRNAs, 3 of which increased and 9 of which decreased, as shown in Figure 1A. Similar analysis between NF and ISC hearts (n = 5) revealed changes in the expression of 16 miRNAs, five of which increased and 11 of which decreased, as shown in Figure 1B. Average miR expression by micro array is shown in Figure 2 for all significant changes in miR expression in NF compared to either IDC or ISC in human heart.

miRNA RT-PCR: Reverse transcription of miRNAs was performed using the TaqMan MicroRNA Reverse Transcription Kit (ABI) according to manufacturer's recommendations. Briefly, 5 ng of miRNA were combined with dNTPs, MultiScribe reverse transcriptase and the primer specific for the target miRNA. The resulting cDNA was diluted 15-fold, and used in PCR reactions. PCR was performed according to manufacturer's recommendations (Applied Biosystems). Briefly, cDNA was combined with the TaqMan assay specific for the target miRNA, and PCR reaction was performed using the ABI7300.

Figure 4 shows RT-PCR validation of miRNA expression in human hearts of 6 non-failing (NF), 5 idiopathic cardiomyopathy (IDC) and 5 ischemic cardiomyopathy (ISC) patients. Data was normalized to miRNA-24, since the expression level of this microRNA did not change. Expression of miR-92 and miR-133b in IDC and ISC failing hearts was significantly decreased when compared to NF hearts, while the expression of miR-100 and miR-195 was significantly increased when compared to NF hearts.

Because the sequences for miR-133a and miR-133b are highly similar, experiments were performed wherein either miR-133a or miR-133b inhibitors or mimics were over-expressed. Data obtained by RT-PCR provided confirmation that detection was specific to each miR. Thus, over-expression of miR-133a demonstrated increased expression of miR-133a with no change in the abundance of miR-133b. The results of the converse experiment yielded the same degree of specificity.

### Example 2. The fetal cardiac gene program can be modulated by regulation of specific miRNAs

Reactivation of the fetal cardiac gene program (FGP) is a characteristic feature of hypertrophied and failing hearts that correlates with impaired cardiac function and poor prognosis. (Kuwahara et al., 2003, EMBO J. 22 (23): 6310-6321) Cardiac alpha-myosin heavy chain (αMyHC), beta-myosin heavy chain (βMyHC), atrial natriuretic factor (ANF), brain natriuretic peptide (BNP), alpha-skeletal actin (Sk α-actin) and calcium ATPase (SERCA) are among genes whose expression is indicative of FGP activation, and are altered in heart failure and may adversely impact on disease progression. For example, beta-adrenergic signaling is known to play an important role in the natural history of dilated cardiomyopathies. Chronic activation of beta-adrenergic receptors (beta₁-AR and beta₂-AR) during periods of cardiac stress ultimately harms the failing heart by mechanisms that include alterations in gene expression. It was previously shown by Sucharov *et al.,* 2006, that stimulation of beta-ARs with isoproterenol (a beta adrenergic receptor agonist) in neonate rat ventricular myocytes causes a "fetal" response in the relative activities of the human cardiac fetal and/or adult gene promoters that includes repression of the human and rat alpha-myosin heavy chain (αMyHC) promoters with simultaneous activation of the human atrial natriuretic peptide (ANP) and rat βMyHC promoters (Sucharov et al., 2006, A beta1-adrenergic receptor CaM kinase II-dependent pathway mediates cardiac myocyte fetal gene induction Am J Physiol Heart Circ Physiol 291: H 1299-H 1308). It was also previously shown that the promoter changes correlate with changes in endogenous gene expression as measured by mRNA expression.

The objective of the experiments described in this example was to determine the effects of expression or inhibition of particular miRNAs on activation of the fetal gene program in the absence and presence of stimulation of beta-adrenergic receptors.

miRIDIAN™ microRNA mimics and inhibitors (Dharmacon) were transfected into neonate rat ventricular myocytes (NRVMs) using the Amaxa technology (Amaxa AG, Cologne, Germany). Product numbers of Dharmacon mimics and inhibitors that were used are as follows: hsa-miR-92 mimic: C-00030-02; based upon double stranded miRNA of the Sanger sequence for miR-92; hsa-miR-92 inhibitor: 1-300030-02, based upon single stranded miRNA of the Sanger-based sequence for miR-92: uauugcacuugucccggccugu (SEQ ID NO: 5). hsa-miR-100 mimic: C-300036-01; based upon double stranded miRNA of the Sanger sequence for miR100; hsa-miR-100 inhibitor: 1-300036-01; based upon single stranded miRNA of the Sanger-based sequence for miR-100: aacccguagauccgaacuugug (SEQ ID NO: 7). hsa-miR-133b mimic: C-300199-01; based upon double stranded miRNA of the Sanger sequence for miR133b; hsa-miR-133b inhibitor: 1-300199-01; based upon single stranded miRNA of the Sanger-based sequence for miR133b: uugguccccuucaaccagcua (SEQ ID NO: 19).

2.4x10⁶ NRVMs were suspended in the appropriate Amaxa solution for cardiac myocytes and combined with 20 micromolar of the mimic or inhibitor. Cells were electroporated using the cardiac myocyte program, which resulted in 95% transfection efficiency for short RNAs. Media containing serum was added to the cells. After 24 hours, the media was replaced with serum-free media. Cells were harvested 72 hours after transfection. A subset of cells was treated with the β-adrenergic agonist isoproterenol (10⁻⁷M) for 48 hours post-transfection.

mRNA was extracted from the harvested cells using TRIZol (Invitrogen). cDNA for polyA containing mRNAs was prepared using the iScript (Bio-Rad) essentially as described by the manufacturer. Typically, 0.1 ng ofcDNA, 12.5 nM of each primer and Power Syber Green PCR Master Mix (ABI) were used in the RT-PCR reactions. Reactions were performed using the ABI7300 system. The primers that were used are presented in Table 2 (below). miRNA cDNAs were synthesized and RT-PCR reaction was performed according to ABI as described in Example 1.

| Table 2: Sequence of primers used for RT-PCR reaction. | |
|---|---|
| All primers are presented in a 5'-3' orientation. | |
| alphaMyHC F | CCTGTCCAGCAGAAAGAGC (SEQ ID NO: 34) |
| alphaMyHC R | CAGGCAAAGTCAAGCATTCATATTTATTGTG (SEQ ID NO: 35) |
| 18S F | GCCGCTAGAGGTGAAATTCTTG (SEQ ID NO: 36) |
| 18S R | CTTTCGCTCTGGTCCGTCTT (SEQ ID NO: 37) |
| BNP F | GGTGCTGCCCCAGATGATT (SEQ ID NO: 38) |
| BNP R | CTGGAGACTGGCTAGGACTTC (SEQ ID NO: 39) |
| SERCA F | GGCCAGATCGCGCTACA (SEQ ID NO: 40) |
| SERCA R | GGGCCAATTAGAGAGCAGGTTT (SEQ ID NO: 41) |
| Sk alpha-actin F | CCACCTACAACAGCATCATGAAGT (SEQ ID NO: 42) |
| Sk alpha-actin R | GACATGACGTTGTTGGCGTACA (SEQ ID NO: 43) |
| betaMyHC F | CGCTCAGTCATGGCGGAT (SEQ ID NO: 44) |
| betaMyHC R | GCCCCAAATGCAGCCAT (SEQ ID NO: 45) |
| ANF F | GCGAAGGTCAAGCTGCTT (SEQ ID NO: 46) |
| ANFR | CTGGGCTCCAATCCTGTCAAT (SEQ ID NO: 47) |

As described above, stimulation of beta-adrenergic receptors with the receptor agonist isoproterenol (ISO) activates the fetal gene program (FGP). ISO-mediated induction of the FGP is manifested by repression of expression of the adult genes, α-myosin heavy chain (αMyHC) and SERCA, and up-regulation of the fetal genes, b-type natriuretic peptide (BNP), atrial natriuretic factor (ANF), skeletal α-actin and β-myosin heavy chain (βMyHC). Figure 5 shows fetal gene program (FGP) expression in neonate cardiac myocytes transfected with miR-92 mimic or miR-92 inhibitor in the absence and presence of isoproterenol. Mimic and inhibitor scrambled miRs (Dharmacon) were used as controls (Con-M and Con-I, respectively). ISO-treated NRVMs transfected with a mimic or inhibitor control exhibited decreased expression of αMyHC and SERCA and increased expression of ANF, BNP, α-skeletal actin, and βMyHC characteristic of activation of the FGP.

Since miR-92 is down-regulated in heart failure, we hypothesized that inhibition of miR-92 (i.e. treatment with a miR-92 inhibitor) would result in induction of the fetal gene program, and that up-regulation of miR-92 (i.e. treatment with a miR-92 mimic) would prevent induction of the fetal gene program. Slight increases in expression of ANF, BNP and α-skeletal actin as well as a decrease in Serca mRNA were observed in cells transfected with the miR-92 mimic as compared to control (Figure 5). However, in general, inhibition or up-regulation of miR-92 had a minimal effect on the regulation of fetal or adult gene expression. These results suggest that down-regulation of miR-92 in heart failure may be involved in other pathways modulating the heart failure phenotype.

Figure 6 shows fetal gene program (FGP) expression in neonate cardiac myocytes transfected with miR-100 mimic or miR-100 inhibitor in the presence and absence of isoproterenol. Mimic and inhibitor scrambled miRs (Con-M and Con-I, respectively, Dharmacon) were used as controls. Transfection of ventricular myocytes with a miR-100 mimic resulted in repression of the adult genes αMyHC and SERCA and enhanced ISO-mediated up-regulation of the fetal genes ANF and βMyHC. Interestingly, down-regulation of miR-100 prevented ISO-mediated repression of αMyHC and SERCA, but did not prevent the ISO-induction of the fetal isoforms (Figure 6), suggesting that inhibition of miR-100 specifically regulates expression of genes involved in ISO-mediated repression of the adult isoforms.

Figure 7 shows fetal gene program (FGP) expression in neonate cardiac myocytes transfected with a miR-133b mimic or miR-133b inhibitor in the presence of absence of isoproterenol. Mimic and inhibitor scrambled miRs (Dharmacon) were used as controls (Con-M and Con-I, respectively). Inhibition of miR-133b resulted in a small but generalized up-regulation of the genes analyzed as compared to control (Con-I). However, up-regulation of miR-133b by transfection with a miR-133b mimic prevented ISO-mediated down-regulation of αMyHC and SERCA, ISO-mediated up-regulation of βMyHC, and reduced ISO-mediated up-regulation of BNP (Figure 7). Over-expression of miR-133b also resulted in repression of skeletal α-actin and βMyHC with no changes in BNP and ANF expression when compared to control (Con-M). These results suggest that changes in miR-133b expression have an important role in the regulation of the fetal gene program similar to the ones presented by Care et al. (Nat Med (2007), Vol. 13(5):613-618), who demonstrated that up-regulation of miR-1333 prevented or reduced α-adrenergic induction of hypertrophic gene program. These findings suggest that the miR-133 family may be a global regulator of gene expression in cardiac disease.

To confirm that each of the miRNA mimics or inhibitors produced upregulation or downregulation of the target miRNA, respectively, mimics and inhibitors for miR-92, miR-100 and miR-133b were transfected into NRVMs as described above. Expression levels of each miRNA were tested by RT-PCR. The results, shown in Figure 8, demonstrate that the miRNA mimics result in an over-expression of the target miRNAs, while the miRNA inhibitors suppress the expression of the specific miRNAs, and this altered level of miR expression is maintained for 72 hours.

### Example 3. miR-133b and cellular hypertrophy

Since inhibition of miR-133b induced expression of the fetal isoforms BNP, skeletal α-actin and ANF, and over-expression of miR-133b blocked ISO-mediated activation of the fetal gene program (Example 2), the effect of over-expression or inhibition of miR-133b on cellular hypertrophy was tested. NRVMs were transfected with mimic and inhibitors as described in Example 2, and stained with an anti α-actinin antibody. Some cells were treated with isoproterenol post-transfection. Immunofluorescence was performed according to Harrison et al. (Molecular & Cellular Biology (2004) Vol. 24(24):10636-10649). Briefly, cells were washed with TBST and fixed with 10% formaldehyde for 20 minutes. Cells were again washed with TBST and incubated with 0.1 % Triton-X for an additional 30 minutes. Cells were then blocked with 1% BSA in TBST for 1 hour followed by 1 hour incubation with 1:500 dilution of the anti α-actinin antibody. Cells were then incubated with a 1:1000 dilution of Alexa 594 anti-mouse antibody and 2µg/ml Hoechst stain for 1 hour. Images were captured at a 40X magnification with a fluorescence microscope (Nikon E800) equipped with a digital camera (Zeiss AxioCam) and Zeiss AxioVision ver. 3.0.6.36 imaging software. Cell surface area of 30 cells from three different fields in each condition was quantified using the Image J software program (NIH).

As shown in Figures 9 and 10, down-regulation of miR-133b induced an increase in cell size, while over-expression of miR-133b dramatically reduced ISO-mediated increases in cardiomyocyte cell size. These results further support the view that miR-133b may be a global regulator of cardiomyocyte hypertrophy.

### Example 4. SAGE approach to miR-based target discovery

The schematic depicted in Figure 11 illustrates the serial analysis of gene expression (SAGE) approach to identifying therapeutic targets for heart failure. Patients with heart failure (HF), associated with a heart failure clinical trial, undergo elective endomyocardial biopsy at three different time points. The first time point is prior to initiation of beta-blocker (BB) therapy and the remaining time points are at 3 and 12 months after initiation of therapy. From each patient, biopsies at each time point are processed for miRNA array analysis, mRNA (Afflymetrix) array analysis, and (targeted) proteomic expression analysis. The unique combination of miR/mRNA/protein expression from the same sample simultaneously represents a unique opportunity to examine changes in gene expression patterns at multiple levels. As miRNA expression affects mRNA expression, which subsequently affects protein expression, here-to-for undefined linkages between control points of regulatory control can be examined. Furthermore, analysis of samples across time from the same patient obviates significant issues related to inter-individual genetic variation. Therapeutic targets may be identified by comparing the levels of gene expression from the same patient at different time points (e.g. 3 months post-therapy vs. pre-therapy, etc.). Gene expression levels may also be correlated with clinical outcome.

### Example 5. Changes in miRNA expression in human patients treated with β-blocker therapy

To further illustrate the importance of miRNAs in heart failure, miRNA expression was assessed in human patients before and after treatment with β-blockers. Serial cardiac biopsies were obtained from five patients at end stage heart failure (prior to treatment), at 3 months post-treatment, and 12 months post-treatment with β-blocker therapy. miRNA array analysis was performed on each of the samples for each of the patients to determine expression levels for several miRNAs, including miR-1, miR-19b, miR-133b, miR-133a, miR-30d, miR-92a, miR-208b, miR-499, and miR-let-7g, which are down-regulated in failing hearts. The expression levels determined in the miRNA array analysis were confirmed by RT-PCR as described in Example 1. The results of the RT-PCR are shown in Figures 12-16. Expression levels of the particular miRNAs were normalized to either miR-370 (right panels) or the small RNA RNU66 (left panels), which were used as controls. miRNA expression was assessed at end stage heart failure (A), 3 months post-treatment (B) and 12 months post-treatment (C). miRNA expression was upregulated in post-treatment samples for the four patients who responded to β-blocker therapy (20, 21, 25 and 34). No up-regulation of the specific miRNAs was observed in the samples obtained from the patient who did not respond to treatment (patient 103). The results demonstrate that the expression level of a subset of miRNAs correlates with the severity of heart failure and can be used as biomarkers to monitor a patient's response to a particular drug therapy as described in Example 4. The dosage or type of drug therapy may be adjusted based on the expression levels of one or more of these miRNAs.

### Example 6. Protein kinase Cε, phosphodiestarase 1A, and calmodulin are targets for miR-92 and miR-133

A 3'UTR analysis of Protein kinase Cε (PKCε) and phosphodiestarase 1A (PDEIA) mRNAs by Target Scan 4.2 (available on the target scan website) revealed possible target sites for miR-92a (previously named miR-92). In order to test if miR-92a targets these two mRNAs, rat neonate cardiac myocytes were transfected with a miR-92a mimic, resulting in over-expression of miR-92a, or a miR-92a inhibitor, resulting in down-regulation of miR-92a. Cells were harvested 72 hours after transfection and cell lysates were analyzed by Western Blot. Down-regulation of miR-92a by the miR-92a inhibitor resulted in an up-regulation of PKCε (Figure 17 A) and PDE1A (Figure 17B) protein levels. Up-regulation of miR-92a by the miR-92a mimc resulted in repression of PKCε (Figure 17A) and PDE1A (Figure 17B) expression.

A similar analysis was performed for the 3'UTR of calmodulin mRNA by Target Scan. The analysis revealed possible target sites for miR-133b. To test whether miR-133b targets calmodulin, rat neonate cardiac myocytes were transfected with a miR-133b mimic (resulting in over-expression of miR-133b) or a miR-133b inhibitor (resulting in down-regulation of miR-133b). Cells were harvested 72 hours after transfection and calmodulin mRNA expression was analyzed by RT-PCR (Figure 18A). Calmodulin protein expression was analyzed by Western Blot (Figure 18 C). Down-regulation of miR-133b by the miR-133b inhibitor resulted in up-regulation of calmodulin mRNA and protein levels, while up-regulation of miR-133b by the miR-133b mimic had a minimal effect on calmodulin expression.

To determine if miR-133b had a direct effect on the calmodulin 3'UTR, rat neonate cardiac cells were co-transfected with miR-133b mimic or miR-133b inhibitor and a construct containing the calmodulin 3'UTR linked to luciferase (Figure 18B). The results of the luciferase assay confirmed the mRNA and protein expression analysis findings. Transfection with the miR-133b mimic did not produce a significant change in luciferase levels. However, transfection of a miR-133b inhibitor resulted in an upregulation of luciferase demonstrating that calmodulin is a direct target of miR-133b.

Taken together, these results show that PKCε and PDE1A are targets of miR-92a, while calmodulin is a target of miR-133b. All three of these targets are relevant to the development of cardiomyopathies leading to heart failure.

It is understood that the disclosed invention is not limited to the particular methodology, protocols and materials described as these may vary. It is also understood that the terminology used herein is for the purposes of describing particular embodiments only and is not intended to limit the scope of the present invention which will be limited only by the appended claims.

### References

Lloyd-Jones, D. M. et al. Lifetime risk for developing congestive heart failure: the Framingham Heart Study. Circulation 106, 3068-72 (2002)
American Heart Association. Heart Disease and Stroke Statistics--2003 Update, American Heart Association, Dallas, Tex., 2003
Bartel, 2004 Cell, 116, 281-297
Ambros, 2003 MicroRNA pathways in flies and worms: growth, death, fat, stress and timing. Cell 113: 673-676
Xu et al., 2003 The Drosophila microRNA Mir-14 suppresses cell death and is required for normal fat metabolism. Curr. Biol. 13:790-795
Landgraf et al, 2007 A Mammalian microRNA Expression Atlas Based on Small RNA Library Sequencing. Cell 129:1401-14
Pfeffer, S. et al., 2004 Identification of virus-encoded microRNAs. Science 304: 734-736
Calin, GA et al., 2004 Human microRNA genes are frequently located at fragile sites and genomic regions involved in cancers. Proc. Natl. Acad. Sci. USA 101: 2999-3004
Calin GA et al., 2002 Frequent deletions and downregulation of micro-RNA genes miR15 and miR16 at 13q14 in chronic lymphocytic leukemia. Proc. Natl. Acad. Sci. USA 99(24): 15524-15529
Care et al., 2007, MicroRNA-133 controls cardiac hypertrophy, Nature Med. 13 (5):613-618
Cheng et al., 2007, MicroRNAs are aberrantly expresses in hypertrophic heart, Am J. Pathol 170: 1831-1840
Sayed et al., 2007, MicroRNAs play an essential role in the development of cardiac hypertrophy, Circ. Res. 100: 416-424
Yang et al., 2007 The muscle-specific microRNA miR-1 regulates cardiac arrhythmogenic potential by targeting GJA1 and KCNJ2, Nature Med. 13 (4): 486-491
van Rooij et al., 2007, Control of stress-dependent cardiac growth and gene expression by a microRNA, Science 316:575-579
van Rooij et al., 2006, A signature pattern of stress-responsive microRNAs that can evoke cardiac hypertrophy and heart failure. PNAS 103:18255-18260
Pfeffer, S. et al., 2004 Identification of virus-encoded microRNAs. Science 304: 734-736
Schena et al., Proc. Natl. Acad. Sci. USA 93:10614 10619, 1996
Heller et al., Proc. Natl. Acad. Sci. USA 94:2150 2155, 1997
Hutvagner et al. 2004, Sequence specific inhibition of small RNA function, PLoS Biology 2(4): 0465-0475
Meister et al. 2004, Sequence-specific inhibition of microRNA- and siRNA-induced RNA silencing, RNA 10: 544-550
Kuwahara et al., 2003, EMBO J. 22 (23): 6310-6321
Sucharov et al., 2006, A beta1-adrenergic receptor CaM kinase II-dependent pathway mediates cardiac myocyte fetal gene induction Am J Physiol Heart Circ Physiol 291: H1299-H1308

## Claims

1. An *in vitro* method of diagnosing ischemic cardiomyopathy in a subject, the method comprising:
a. determining the level of expression of one or more miRNAs in a biological sample from the subject, wherein said one or more miRNAs is selected from the group consisting of hsa-miR-199a (SEQ ID NO: 25), hsa-miR-195 (SEQ ID NO: 4), hsa-miR-100 (SEQ ID NO: 7), hsa-miR-342 (SEQ ID NO: 14), hsa-miR-28 (SEQ ID NO: 24), hsa-miR-26b (SEQ ID NO: 30), hsa-miR-92a (SEQ ID NO: 5), hsa-miR-221 (SEQ ID NO: 17), hsa-miR-486 (SEQ ID NO: 11), hsa-miR-133a (SEQ ID NO: 15), hsa-miR-150 (SEQ ID NO: 12), hsa-miR-422b (SEQ ID NO: 16), hsa-let-7f (SEQ ID NO: 18), hsa-let-7a (SEQ ID NO: 22), hsa-miR-1 (SEQ ID NO: 23), hsa-miR-222 (SEQ ID NO: 20), hsa-let-7c (SEQ ID NO: 28), hsa-miR-133b (SEQ ID NO: 19), hsa-miR-224 (SEQ ID NO: 21), hsa-miR-484 (SEQ ID NO: 29), and hsa-let-7d (SEQ ID NO: 31); and
b. comparing the level of expression of one or more miRNAs in said biological sample to the level of one or more miRNAs in a standard sample,
wherein a difference in the level of expression in at least one or more miRNAs in said biological sample compared to the level of one or more miRNAs in said standard sample is diagnostic for ischemic cardiomyopathy.

2. The method of claim 1, wherein an increase in the level of any one of hsa-miR-199a (SEQ ID NO: 25), hsa-miR-195 (SEQ ID NO: 4), hsa-miR-100 (SEQ ID NO: 7), hsa-miR-342 (SEQ ID NO: 14), hsa-miR-26b (SEQ ID NO: 30), and hsa-miR-28 (SEQ ID NO: 24) is diagnostic of ischemic cardiomyopathy.

3. The method of claim 1, wherein a decrease in the level of any one of hsa-miR-92a (SEQ ID NO: 5), hsa-miR-221 (SEQ ID NO: 17), hsa-miR-486 (SEQ ID NO: 11), hsa-miR-133a (SEQ ID NO: 15), hsa-miR-150 (SEQ ID NO: 12), hsa-miR-422b (SEQ ID NO: 16), hsa-let-7f (SEQ ID NO: 18), hsa-let-7a (SEQ ID NO: 22), hsa-miR-1 (SEQ ID NO: 23), hsa-miR-222 (SEQ ID NO: 20), hsa-1et-7c (SEQ ID NO: 28), hsa-miR-133b (SEQ ID NO: 19), hsa-miR-224 (SEQ ID NO: 21), hsa-miR-484 (SEQ ID NO: 29), and hsa-let-7d (SEQ ID NO: 31) is diagnostic of ischemic cardiomyopathy.

## Patentansprüche

1. In vitro Verfahren zur Diagnose ischämischer Kardiomyopathie bei einer Person, wobei das Verfahren die folgenden Schritte umfasst:
a. Bestimmung des Expressionslevels einer oder mehrerer miRNAs in einer biologischen Probe von der Person, wobei die eine oder mehreren miRNAs ausgewählt ist aus der Gruppe bestehend aus hsa-miR-199a (SEQ ID NO: 25), hsa-miR-195 (SEQ ID NO: 4), hsa-miR-100 (SEQ ID NO: 7), hsa-miR-342 (SEQ ID NO: 14), hsa-miR-28 (SEQ ID NO: 24), hsa-miR-26b (SEQ ID NO: 30), hsa-miR-92a (SEQ ID NO: 5), hsa-miR-221 (SEQ ID NO: 17), hsa-miR-486 (SEQ ID NO: 11), hsa-miR-133a (SEQ ID NO: 15), hsa-miR-150 (SEQ ID NO: 12), hsa-miR-422b (SEQ ID NO: 16), hsa-let-7f (SEQ ID NO: 18), hsa-let-7a (SEQ ID NO: 22), hsa-miR-1 (SEQ ID NO: 23), hsa-miR-222 (SEQ ID NO: 20), hsa-let-7c (SEQ ID NO: 28), hsa-miR-133b (SEQ ID NO: 19), hsa-miR-224 (SEQ ID NO: 21), hsa-miR-484 (SEQ ID NO: 29) und hsa-let-7d (SEQ ID NO: 31); und
b. Vergleich des Expressionslevels einer oder mehrerer miRNAs in der biologischen Probe mit dem Level einer oder mehrerer miRNAs in einer Standardprobe,
wobei ein Unterschied im Expressionslevel mindestens einer oder mehrerer miRNAs in der biologischen Probe verglichen mit dem Level einer oder mehrerer miRNAs in der Standardprobe diagnostisch für ischämische Kardiomyopathie ist.

2. Verfahren gemäß Anspruch 1, wobei eine Erhöhung im Level von irgendeiner von hsa-miR-199a (SEQ ID NO: 25), hsa-miR-195 (SEQ ID NO: 4), hsa-miR-100 (SEQ ID NO: 7), hsa-miR-342 (SEQ ID NO: 14), hsa-miR-26b (SEQ ID NO: 30) und hsa-miR-28 (SEQ ID NO: 24) diagnostisch für ischämische Kardiomyopathie ist.

3. Verfahren gemäß Anspruch 1, wobei eine Verminderung im Level von irgendeiner von hsa-miR-92a (SEQ ID NO: 5), hsa-miR-221 (SEQ ID NO: 17), hsa-miR-486 (SEQ ID NO: 11), hsa-miR-133a (SEQ ID NO: 15), hsa-miR-150 (SEQ ID NO: 12), hsa-miR-422b (SEQ ID NO: 16), hsa-let-7f (SEQ ID NO: 18), hsa-let-7a (SEQ ID NO: 22), hsa-miR-1 (SEQ ID NO: 23), hsa-miR-222 (SEQ ID NO: 20), hsa-let-7c (SEQ ID NO: 28), hsa-miR-133b (SEQ ID NO: 19), hsa-miR-224 (SEQ ID NO: 21), hsa-miR-484 (SEQ ID NO: 29) und hsa-let-7d (SEQ ID NO: 31) diagnostisch für ischämische Kardiomyopathie ist.

## Revendications

1. Procédé in vitro de diagnostic d'une cardiomyopathie ischémique chez un sujet, le procédé comprenant :
a. la détermination du taux d'expression d'un ou de plusieurs miARN dans un échantillon biologique provenant du sujet, dans lequel ledit un ou plusieurs miARN est choisi dans le groupe constitué par hsa-miR-199a (SEQ ID NO: 25), hsa-miR-195 (SEQ ID NO: 4), hsa-miR-100 (SEQ ID NO: 7), hsa-miR-342 (SEQ ID NO: 14), hsa-miR-28 (SEQ ID NO: 24), hsa-miR-26b (SEQ ID NO: 30), hsa-miR-92a (SEQ ID NO: 5), hsa-miR-221 (SEQ ID NO: 17), hsamiR-486 (SEQ ID NO: 11), hsa-miR-133a (SEQ ID NO: 15), hsa-miR-150 (SEQ ID NO: 12), hsa-miR-422b (SEQ ID NO: 16), hsa-let-7f (SEQ ID NO: 18), hsa-let-7a (SEQ ID NO: 22), hsa-miR-1 (SEQ ID NO: 23), hsa-miR-222 (SEQ ID NO: 20), hsa-let-7c (SEQ ID NO: 28), hsa-miR-133b (SEQ ID NO: 19), hsa-miR-224 (SEQ ID NO: 21), hsa-miR-484 (SEQ ID NO: 29), et hsa-let-7d (SEQ ID NO: 31) ; et
b. la comparaison du taux d'expression d'un ou de plusieurs miARN dans ledit échantillon biologique au taux d'un ou de plusieurs miARN dans un échantillon de référence,
dans lequel une différence dans le taux d'expression dans au moins un ou plusieurs miARN dans ledit échantillon biologique par rapport au taux d'un ou de plusieurs miARN dans ledit échantillon de référence est un diagnostic d'une cardiomyopathie ischémique.

2. Procédé selon la revendication 1, dans lequel une augmentation du taux de l'un quelconque de hsa-miR-199a (SEQ ID NO: 25), hsa-miR-195 (SEQ ID NO: 4), hsa-miR-100 (SEQ ID NO: 7), hsa-miR-342 (SEQ ID NO: 14), hsa-miR-26b (SEQ ID NO: 30), et hsa-miR-28 (SEQ ID NO: 24) est un diagnostic d'une cardiomyopathie ischémique.

3. Procédé selon la revendication 1, dans lequel une diminution du taux de l'un quelconque de hsa-miR-92a (SEQ ID NO: 5), hsa-miR-221 (SEQ ID NO: 17), hsa-miR-486 (SEQ ID NO: 11), hsa-miR-133a (SEQ ID NO: 15), hsa-miR-150 (SEQ ID NO: 12), hsa-miR-422b (SEQ ID NO: 16), hsa-let-7f (SEQ ID NO: 18), hsa-let-7a (SEQ ID NO: 22), hsa-miR-1 (SEQ ID NO: 23), hsa-miR-222 (SEQ ID NO: 20), hsa-let-7c (SEQ ID NO: 28), hsa-miR-133b (SEQ ID NO: 19), hsa-miR-224 (SEQ ID NO: 21), hsa-miR-484 (SEQ ID NO: 29), et hsa-let-7d (SEQ ID NO: 31) est un diagnostic d'une cardiomyopathie ischémique.
